(19) Europäisches Patentamt
European Patent Office
Office européen des brevets

(11) EP 2 702 082 B1

(12) **FASCICULE DE BREVET EUROPEEN**

(45) Date de publication et mention de la délivrance du brevet:
**13.09.2017 Bulletin 2017/37**

(21) Numéro de dépôt: **12724085.1**

(22) Date de dépôt: **27.04.2012**

(51) Int Cl.:
***C08F 289/00*** *(2006.01)* ***C08L 91/00*** *(2006.01)*
***C08L 101/12*** *(2006.01)*

(86) Numéro de dépôt international:
**PCT/EP2012/057813**

(87) Numéro de publication internationale:
**WO 2012/146741 (01.11.2012 Gazette 2012/44)**

(54) **CORPS GRAS COPOLYMÉRISÉ, SON PROCÉDÉ DE PRÉPARATION ET SES APPLICATIONS**

COPOLYMERISIERTER FETTSÄUREKÖRPER, HERSTELLUNGSVERFAHREN DAFÜR UND VERWENDUNGEN DAVON

COPOLYMERISED FATTY BODY, PREPARATION METHOD THEREOF AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.04.2011 FR 1153636**
**28.04.2011 FR 1153634**

(43) Date de publication de la demande:
**05.03.2014 Bulletin 2014/10**

(60) Demande divisionnaire:
**17176775.9**

(73) Titulaire: **Rhodia Opérations**
**93306 Aubervilliers (FR)**

(72) Inventeurs:
- **BALASTRE, Marc**
**75020 Paris (FR)**
- **VUONG, Chi-Thanh**
**77185 Lognes (FR)**

(74) Mandataire: **Cardon, Flavie**
**RHODIA OPERATIONS**
**Direction de la Propriété Industrielle**
**40, rue de la Haie-Coq**
**93306 Aubervilliers Cedex (FR)**

(56) Documents cités:
**WO-A1-2009/119423** **DE-A1- 2 620 309**
**GB-A- 1 564 542**

EP 2 702 082 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne la copolymérisation d'un corps gras pour notamment modifier ses propriétés rhéologiques ; le copolymère ainsi obtenu et son utilisation pour modifier les propriétés notamment rhéologiques d'un milieu non-aqueux.

Les formulations à base de milieux non aqueux, notamment apolaires, par exemple d'huiles, sont utilisées dans de nombreux domaines tels que l'agrochimie, la cosmétique, l'industrie pétrolière, les compositions lubrifiantes, les compositions de revêtements, la pharmacie, etc.

Il est généralement nécessaire de modifier la rhéologie de ces milieux pour obtenir les propriétés recherchées. Contrairement aux formulations aqueuses pour lesquelles existent de nombreux agents rhéologiques performants (épaississant, dispersant...), il est plus difficile de trouver des additifs rhéologiques appropriés sur une large gamme de température pour des milieux non-aqueux.

Quelques technologies utilisant notamment des matériaux inorganiques (fumée de silice, argiles modifiées telles que les bentonites) sont déjà mises en oeuvre pour modifier la rhéologie de milieux non aqueux, notamment apolaires, en particulier les huiles. De façon générale, selon la fonctionnalité recherchée et l'application visée il est nécessaire de mettre au point de façon très précise des produits issus de chacune de ces technologies. Néanmoins, pour toutes ces applications, il serait utile de pouvoir proposer un produit aisé de mise en oeuvre, ayant un prix raisonnable et conférant aux milieux non aqueux, notamment apolaires, en particulier aux huiles un comportement pseudo plastique avec présence d'un seuil rhéologique, et ce sur une large gamme de température (y compris dans des conditions de stockage drastique, notamment en termes de durée et de température).

Il y a donc un intérêt à fournir un composé et un procédé permettant de modifier la rhéologie des milieux non aqueux, notamment apolaires, en particulier des huiles, de mise en oeuvre aisé, ayant un prix raisonnable et conférant audits milieux non aqueux, notamment apolaires, en particulier audites huiles un comportement pseudo plastique avec présence d'un seuil rhéologique, et ce sur une large gamme de température.

Un objectif de la présente invention est de fournir un copolymère permettant notamment de modifier les propriétés, en particulier les propriétés rhéologiques (présence d'un seuil rhéologique et/ou modification de la viscosité, avantageusement augmentation de la viscosité, et/ou modifications des propriétés de gélification), d'un milieu non aqueux, notamment apolaire, en particulier d'huiles.

Un autre objectif de l'invention est de fournir un procédé simple de modification de la rhéologie (présence d'un seuil rhéologique et/ou modification de la viscosité, notamment augmentation de la viscosité, et/ou modification des propriétés de gélification) de milieux non aqueux, notamment apolaires, en particulier d'huiles.

Un autre objectif de la présente invention concerne un procédé de modification des propriétés, notamment les propriétés rhéologiques (présence d'un seuil et/ou modification de la viscosité, notamment augmentation de la viscosité, et/ou modification des propriétés de gélification), d'un corps gras.

D'autres objectifs apparaîtront à la lecture de la description de la présente invention.

Dans le cadre de l'invention on entend par « corps gras », tout composé contenant une chaine aliphatique, linéaire ou ramifiée, comprenant au moins 4 atomes de carbone, par exemple au moins 6 atomes de carbone, par exemple au moins 8 atomes de carbone, par exemple au moins 10 atomes de carbone. Il peut s'agir d'un composé lipophile ou amphiphile. Par exemple, le corps gras peut être choisi parmi les huiles, ou leurs dérivés, liquides à températures ambiantes, notamment entre 15 et 30°C, par exemple à 25°C; les graisses, ou leurs dérivés, pâteuses ou solides à températures ambiantes, notamment entre 15 et 30°C, par exemple à 25°C ; les cires, ou leurs dérivés, solides à température ambiante, notamment entre 15 et 30°C, par exemple à 25°C.

Le corps gras selon l'invention est généralement immiscible dans l'eau. Un composé est dit immiscible dans l'eau si moins de 3%, de préférence moins de 2%, par exemple moins de 1% en poids de ce composé est sous forme solubilisée dans l'eau.

Au sens de l'invention, un corps gras susceptible de se disperser dans l'eau, par exemple susceptible de générer des micelles dans l'eau, n'est pas considéré comme miscible à l'eau.

Dans le cadre de l'invention on entend par « milieu non aqueux » un milieu significativement exempt d'eau, notamment comprenant moins de 10% en poids d'eau, par exemple moins de 5% en poids d'eau, par exemple moins de 3 % en poids d'eau, de préférence moins de 1% en poids d'eau.

Le milieu non aqueux selon l'invention est notamment utilisable pour la préparation de compositions dans les domaines de la cosmétique, de l'agrochimie, de la pharmacie, de l'industrie pétrolière, de l'industrie automobile, dans le domaine des encres, des revêtements, etc.

Il doit être compris que le milieu non aqueux selon l'invention peut représenter tout ou partie des compositions dont on entend modifier les propriétés, en particulier rhéologiques.

Selon une première variante, la composition peut comprendre uniquement un milieu non aqueux selon l'invention, et être donc exempte d'une phase aqueuse. Dans ce cas, le milieu non aqueux selon l'invention représente l'ensemble de la composition dont on entend modifier les propriétés, en particulier rhéologiques.

Selon une deuxième variante, la composition peut comprendre, outre le milieu non aqueux selon l'invention, une phase aqueuse ou un milieu non aqueux de nature différente du milieu non aqueux dont on entend modifier les propriétés, notamment rhéologiques. Cette variante correspond par exemple aux compositions de type émulsion (directe, inverse ou multiple). Dans ce cas, le milieu non aqueux selon l'invention représente une partie seulement de la composition dont on entend modifier les propriétés, en particulier rhéologiques. Bien entendu, selon cette deuxième variante, la modification des propriétés, notamment rhéologiques, du milieu non aqueux selon l'invention peut induire une modification des propriétés, notamment rhéologiques, de l'ensemble de la composition incorporant ce milieu non aqueux. Par exemple, lorsque la composition dont on entend modifier les propriétés, en particulier rhéologiques, se présente sous la forme d'une émulsion eau-dans-huile, la modification des propriétés, en particulier rhéologiques, du milieu non aqueux (huile) peut avantageusement entrainer une modification des propriétés, en particulier rhéologiques, de l'ensemble de l'émulsion, c'est-à-dire du milieu continu non aqueux mais aussi du milieu aqueux émulsionné.

L'invention concerne ainsi une composition émulsifiable par mélange avec de l'eau telle que définie en revendication 1.

De façon préférée, le copolymère est un copolymère dont le squelette est obtenu par polymérisation radicalaire :

- d'un corps gras (A) comprenant des insaturations tel que défini en revendication 1; et
- d'au moins un monomère (B) comprenant au moins une fonction polymérisable par polymérisation radicalaire et comprenant au moins une chaîne alkyle, linéaire ou ramifiée comprenant de 16 à 44 atomes de carbone, plus préférentiellement au moins 18 atomes de carbones, par exemple au moins 20 atomes de carbone, notamment 22 atomes de carbone ou 44 atomes de carbone.

Il est à noter que, dans le cadre de la présente invention, le monomère (B) peut comprendre plusieurs, notamment 2, chaines alkyles telles que définies précédemment.

Par exemple, il est possible d'utiliser un monomère de type dialkylacrylamide, par exemple dioctylacrylamide, ou encore un dialkylstyrène.

Dans le cadre de l'invention, l'expression « entre x et y » doit être comprise comme incluant les valeurs x et y. Selon l'invention, cette expression signifie également de x à y.

Le corps gras (A) est une huile végétale ou une huile d'origine végétale par exemple choisie parmi :

- les triglycérides d'acides gras saturés ou insaturés comprenant au moins 12 atomes de carbone et de préférence de 14 à 22 atomes de carbone ; il peut s'agir de triglycérides naturels, tels que les huiles végétales ou d'origine végétales du type huile de colza, huile de soja, huile d'arachide, huile de beurre, huile de graine de coton, huile de lin, huile de noix de coco, huile d'olive, huile de palme, huile de pépin de raisin, huile de poisson, huile de ricin, huile de coprah ;
- les esters des triglycérides d'acides gras saturés ou insaturés comprenant au moins 12 atomes de carbone et de préférence de 14 à 22 atomes de carbone, en particulier tels que définis précédemment, et notamment leurs esters méthyliques et éthyliques ;

ou une huile animale ou d'origine animale, par exemple une huile de poisson ;
ou leurs mélanges.

De manière avantageuse, le corps gras (A) peut être une huile végétale ou une huile d'origine végétale choisie par exemple parmi l'huile de colza, l'huile de soja, l'huile de maïs, l'huile de ricin, l'huile d'arachide, l'huile de beurre, l'huile de graine de coton, l'huile de lin, l'huile de noix de coco, l'huile d'olive, l'huile de palme, l'huile de pépin de raisin, l'huile de coprah et leurs mélanges. L'huile de colza, notamment, est bien adaptée à l'invention, de même que les huiles de ricin, de maïs et de soja.

Selon un mode de réalisation, le corps gras (A) peut être choisi parmi l'huile de ricin et l'huile de colza.

De préférence, le corps gras (A) est l'huile de ricin.

Pour les monomères (B) selon l'invention, on entend généralement par fonction polymérisable toute fonction susceptible de polymériser par voie radicalaire.

Ces fonctions sont bien connues de l'homme du métier. A titre illustratif, il peut notamment s'agir d'une fonction choisie parmi les fonctions acrylate, méthacrylate, acrylamide, méthacrylamide, vinyl, notamment, allyle ou vinyléther, et styrène.

En particulier, le monomère (B) peut être choisi parmi :

- les acrylates d'alkyle ;
- les méthacrylates d'alkyle ;
- les acrylamides d'alkyle ;
- les méthacrylamides d'alkyle ;
- les vinyles d'alkyle, notamment les allyles d'alkyles ou les vinyléthers d'alkyle ; et

- les styrènes d'alkyle ;

dans lesquels l'alkyle est une chaîne, linéaire ou ramifiée, comprenant de préférence de 16 à 44 atomes de carbone, plus préférentiellement au moins 18 atomes de carbones, par exemple au moins 20 atomes de carbone, notamment 22 atomes de carbone ou 44 atomes de carbone.

De préférence, le monomère (B) est choisi parmi :

- les acrylates d'alkyle ;
- les méthacrylates d'alkyle ;
- les acrylamides d'alkyle ;
- les méthacrylamides d'alkyle ;

dans lesquels l'alkyle est une chaine, linéaire ou ramifiée, comprenant de préférence de 16 à 44 atomes de carbone, plus préférentiellement au moins 18 atomes de carbones, par exemple au moins 20 atomes de carbone, notamment 22 atomes de carbone ou 44 atomes de carbone.

Plus préférentiellement, le monomère (B) est choisi parmi :

- les acrylates d'alkyle ;
- les méthacrylates d'alkyle ;

dans lesquels l'alkyle est une chaine, linéaire ou ramifiée, comprenant de préférence de 16 à 44 atomes de carbone, plus préférentiellement au moins 18 atomes de carbone, par exemple au moins 20 atomes de carbone, notamment 22 atomes de carbone ou 44 atomes de carbone.

De manière préférée, le monomère (B) peut être choisi parmi :

- les acrylates ou méthacrylates d'alkyle, dans lesquels l'alkyle est une chaine, linéaire ou ramifiée, comprenant de préférence de 16 à 44 atomes de carbone, plus préférentiellement au moins 18 atomes de carbones, par exemple au moins 20 atomes de carbone, notamment 22 atomes de carbone ou 44 atomes de carbone ; et en particulier parmi les acrylates ou méthacrylates d'alcool aliphatiques en $C_3$-$C_{30}$ poly(éthoxylés et/ou propoxylés), de préférence en $C_{16}$-$C_{30}$, plus préférentiellement au moins en $C_{18}$, par exemple au moins en $C_{22}$, dont la partie aliphatique est le cas échéant substituée par un ou plusieurs hydroxyle(s) de préférence en extrémité de groupe aliphatique ;
- les acrylamides ou méthacrylamide d'alkyle, dans lesquels l'alkyle est une chaine, linéaire ou ramifiée, comprenant de préférence de 16 à 44 atomes de carbone, plus préférentiellement au moins 18 atomes de carbones, par exemple au moins 20 atomes de carbone, notamment 22 atomes de carbone ou 44 atomes de carbone; et en particulier choisi parmi les acrylamides ou méthacrylamide d'alcool aliphatiques en $C_3$-$C_{30}$ poly(éthoxylés et/ou propoxylés), de préférence en $C_{16}$-$C_{30}$, plus préférentiellement au moins en $C_{18}$, par exemple au moins en $C_{22}$, dont la partie aliphatique est le cas échéant substituée par un ou plusieurs hydroxyle(s) de préférence en bout extrémité de groupe aliphatique ;
- les styrènes d'alkyle, dans lesquels l'alkyle est une chaine, linéaire ou ramifiée, comprenant de préférence de 16 à 44 atomes de carbone, plus préférentiellement au moins 18 atomes de carbone, par exemple au moins 20 atomes de carbone, notamment 22 atomes de carbone ou 44 atomes de carbone et ses dérivés par exemple comprenant des fonctions halogénées et/ou des fonctions hydroxyles et/ou des fonctions amines; de préférence le chlorure de vinyl benzyle, et le styrène comprenant une chaîne alkyle, ayant de préférence de 16 à 44 atomes de carbone, de préférence située en position para ;
- les vinyles d'alkyle, dans lesquels l'alkyle est une chaine, linéaire ou ramifiée, comprenant notamment de 16 à 44 atomes de carbone, de préférence au moins 18 atomes de carbone, par exemple au moins 20 atomes de carbone ; et en particulier choisi parmi les esters d'alcool allylique dont la chaine alkyle en extrémité de la fonction ester comprend notamment de 16 à 44 atomes de carbone, de préférence au moins 18 atomes de carbone, par exemple au moins 22 atomes de carbone ; ou
- leurs mélanges.

Comme indiqué précédemment, le monomère (B) peut comprendre plusieurs, et notamment deux, chaines alkyles, avec chacune des chaines alkyles étant telle que définie ci-dessus.

De façon particulièrement préférée, le monomère (B) est choisi parmi un acrylate d'alkyle dans lequel la chaine alkyle comprend 22 atomes de carbone, en particulier l'acrylate de béhényle ou un acrylate d'alkyle dans lesquel la chaine alkyle comprend 44 atomes de carbone.

De façon particulièrement préférée, le monomère B est l'acrylate de béhényle.

Selon l'invention au moins un monomère (C) peut en outre être mis en oeuvre pour la préparation du copolymère

selon l'invention. Le monomère (C) est choisi parmi les monomères $C_N$ neutres ; les monomères $C_A$ anioniques ou potentiellement anioniques ; les monomères $C_C$ cationiques ou potentiellement cationiques ; les monomères $C_z$ zwittérioniques ; les monomères Cp hydrophobes ; et leurs mélanges.

On entend par « monomères anioniques ou potentiellement anioniques » des monomères qui comprennent au moins un groupe anionique ou potentiellement anionique. Les groupes anioniques sont des groupes qui présentent au moins une charge négative (généralement associée à un ou plusieurs cations comme des cations de composés alcalins ou alcalino-terreux, par exemple le sodium, ou à un ou plusieurs composés cationiques comme l'ammonium), quel que soit le pH du milieu où est présent le copolymère. Les groupes potentiellement anioniques sont des groupes qui peuvent être neutres ou présenter au moins une charge négative selon le pH du milieu où est présent le copolymère. On entend par « monomères cationiques ou potentiellement cationiques » des monomères qui comprennent au moins un groupe cationique ou potentiellement cationique. Les groupes cationiques sont des groupes qui présentent au moins une charge positive (généralement associée à un ou plusieurs anions comme l'ion chlorure, l'ion bromure, un groupe sulfate, un groupe méthylsulfate), quel que soit le pH du milieu où est présent le copolymère. Les groupes potentiellement cationiques sont des groupes qui peuvent être neutres ou présenter au moins une charge positive selon le pH du milieu où est présent le copolymère.
Par « groupes neutres » on entend des groupes qui ne présentent pas de charge quel que soit le pH du milieu où est présent le copolymère.

Les monomères $C_N$ neutres peuvent notamment être choisis parmi les monomères suivants :

- les hydroxyalkylesters d'acides $\alpha$-$\beta$ éthyléniquement insaturés de péréfence l'acrylate d'hydroxyéthyle, le méthacrylate d'hydroxyéthyle et l'acrylate d'hydroxypropyle ;
- les amides $\alpha$-$\beta$ éthyléniquement insaturés de préférence l'acrylamide, le méthacrylamide, le diméthylacrylamide et l'acrylamide d'hydroxyméthyle ;
- les monomères $\alpha$-$\beta$ éthyléniquement insaturés comportant un segment polyoxyalkylène hydrosoluble avec ou sans une chaîne alkyle de préférence l'acrylate ou le méthacrylate de polyéthylène glycol, avec ou sans chaîne alkyle, de masse moléculaire comprise entre 350 à 5 000 g/mol (étant entendu que cette valeur ne tient pas compte de la chaîne alkyle éventuelle) ;
- l'alcool vinylique ;
- les vinyl-lactames ;
- les monomères $\alpha$-$\beta$ éthyléniquement insaturés uréido de préférence le méthacrylamido de 2-imidazolidinone éthyle ;
- la vinylpyrrolidone ;
- leurs mélanges.

De préférence les monomères $C_N$ sont choisis parmi les (meth)acrylamides, notamment l'acrylamide, le méthacrylamide, le diméthylacrylamide et l'acrylamide d'hydroxyméthyle, la vinylpyrrolidone, l'acrylate d'hydroxyéthyle et le méthacrylate de polyéthylène glycol, avec ou sans chaîne alkyle, de masse moléculaire comprise entre 350 à 5 000g/mol (étant entendu que cette valeur ne tient pas compte de la chaîne alkyle éventuelle).

Les monomères $C_A$ anioniques ou potentiellement anioniques peuvent être choisis parmi les monomères suivants :

- des monomères possédant au moins une fonction carboxylique, par exemple les acides carboxyliques $\alpha$,$\beta$ éthyléniquement insaturés, les anhydrides correspondants et leurs sels hydrosolubles de préférence les acides ou anhydrides acrylique, méthacrylique, maléique, l'acide fumarique, l'acide itaconique, le N-méthacryloyl alanine, le N-acryloylglycine et leurs sels hydrosolubles ;
- des monomères possédant au moins une fonction sulfate ou sulfonate ou une fonction acide correspondante de préférence le 2-sulfooxyethyl méthacrylate, l'acide vinylbenzène sulfonique, l'acide allyl sulfonique, le 2-acrylamido-2méthylpropane sulfonique, l'acrylate ou le méthacrylate de sulfoethyle, l'acrylate ou le méthacrylate de sulfopropyle et leurs sels hydrosolubles ;
- des monomères possédant au moins une fonction phosphonate ou phosphate ou une fonction acide correspondante, de préférence l'acide vinylphosphonique, les esters de phosphates éthyléniquement insaturés ;
- leurs mélanges.

Les monomères $C_A$ particulièrement préférés sont l'acide acrylique et l'acide méthacrylique.

Les monomères $C_C$ cationiques ou potentiellement cationiques peuvent être choisis parmi :

- les monomères avec une fonction amines secondaires, tertiaires ou quaternaires de préférence méthacrylamide de propyldimethylamine, la 4-vinyl aniline et le chlorure de diallyldiméthylamonium (DADMAC) ;
- leurs mélanges.

Les monomères $C_Z$ zwittérioniques c'est-à-dire des monomères comportant deux charges opposées peuvent être choisis parmi :

- les alkylsulfonates ou carboxylates ou phosphonates de dialkylammonium alkyl acrylates ou méthacrylates, acrylamido ou méthacrylamido ;
- les monomères bétaïnes hétérocycliques ;
- les alkylsulfonates ou carboxylates ou phosphonates de dialkylammonium alkyl allyliques ;
- les phosphobétaines de formules :

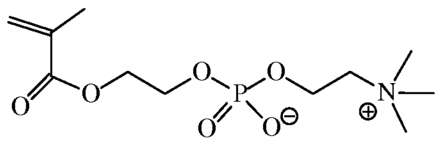

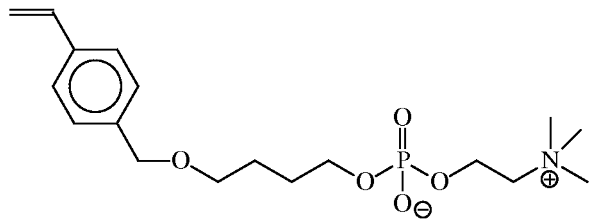

- les bétaines issues d'acétals cycliques.

De façon particulièrement préférée, les monomères $C_z$ sont choisis parmi la diméthyl(méthacrylamidopropyl)(3-sulfopropyl)ammonium bétaïne (SPP) et le 1-(3-sulfopropyl)-2-vinyl-pyridinium-bétaïne (SPV) .

Les monomères $C_p$ sont choisis parmi :

- les acrylates d'alkyle ;
  - les méthacrylates d'alkyle ;
  - les acrylamides d'alkyle ;
  - les méthacrylamides d'alkyle ;
  - les vinyles d'alkyle, notamment les allyles d'alkyles ou les vinyléthers d'alkyle ; et
  - les styrènes d'alkyle ;

dans lesquels l'alkyle est une chaîne, linéaire ou ramifiée, comprenant de préférence de 1 à 44 atomes de carbone, plus préférentiellement de 1 à 22 atomes de carbone.

De préférence les monomères Cp sont choisis parmi :

- les acrylates ou méthacrylates d'alkyle, dans lesquels l'alkyle est une chaine, linéaire ou ramifiée, comprenant de préférence de 1 à 44 atomes de carbone, de préférence de 1 à 22 atomes de carbone ; et en particulier parmi les acrylates ou méthacrylates d'alcool aliphatiques en $C_3$-$C_{30}$ poly(éthoxylés et/ou propoxylés), de préférence en $C_{16}$-$C_{30}$, plus préférentiellement au moins en $C_{18}$, par exemple au moins en $C_{22}$, dont la partie aliphatique est le cas échéant substituée par un ou plusieurs hydroxyle(s) de préférence en extrémité de groupe aliphatique ;
- les acrylamides ou méthacrylamide d'alkyle, dans lesquels l'alkyle est une chaine, linéaire ou ramifiée, comprenant de préférence de 1 à 44 atomes de carbone, plus préférentiellement de 1 à 22 atomes de carbone ; et en particulier choisi parmi les acrylamides ou méthacrylamide d'alcool aliphatiques en $C_3$-$C_{30}$ poly(éthoxylés et/ou propoxylés), de préférence en $C_{16}$-$C_{30}$, plus préférentiellement au moins en $C_{18}$, par exemple au moins en $C_{22}$, dont la partie aliphatique est le cas échéant substituée par un ou plusieurs hydroxyle(s) de préférence en bout extrémité de groupe aliphatique ;
- les styrènes d'alkyle, dans lesquels l'alkyle est une chaine, linéaire ou ramifiée, comprenant de préférence de 1 à 44 atomes de carbone, plus préférentiellement de 1 à 22 atomes de carbone et ses dérivés par exemple comprenant des fonctions halogénées et/ou des fonctions hydroxyles et/ou des fonctions amines ; de préférence le chlorure de vinyl benzyle, et le styrène comprenant une chaîne alkyle, ayant de préférence de 1 à 44 atomes de carbone, de préférence de 1 à 22 atomes de carbone, de préférence située en position para ;

- les vinyles d'alkyle, dans lesquels l'alkyle est une chaine, linéaire ou ramifiée, comprenant notamment de 1 à 44 atomes de carbone, de préférence de 1 à 22 atomes de carbone ; et en particulier choisi parmi les esters d'alcool allylique dont la chaine alkyle en extrémité de la fonction ester comprend notamment de 1 à 44 atomes de carbone, de préférence de 1 à 22 atomes de carbone ;
- leurs mélanges.

Les monomères Cp préférés sont l'acrylate de 2-éthylhexyle et le styrène.

Selon l'invention, le taux de greffage du corps gras (A) dans le copolymère peut atteindre 100%, par exemple 80%, et de préférence être compris entre 5 et 60%. Le taux de greffage est calculé selon l'une des formules suivantes (eq1),ou (eq3) selon que le corps gras (A) comprend respectivement uniquement des insaturations (eq1), ou à la fois des insaturations et des fonctions hydroxyles (eq3):

$$taux\ de\ greffage = \left(\frac{A2}{A1}\right) \quad \text{(eq1)}$$

$$taux\ de\ greffage = \left(\frac{A2}{A1}\right) + \left(\frac{B2}{B1}\right) \quad \text{(eq3)}$$

dans laquelle :

$$\left(\frac{A2}{A1}\right) = \left(\frac{nombre\ d'insaturations\ polymérisées}{nombre\ d'insaturations\ initiales}\right)$$

$$\left(\frac{B2}{B1}\right) = \left(\frac{nombre\ de\ fonctions\ hydroxyles\ substituées}{nombre\ de\ fonctions\ hydroxyles\ initiales}\right)$$

Bien entendu, le taux de greffage peut varier en fonction de la nature du corps gras, et notamment en fonction du nombre d'insaturations et/ou de fonctions hydroxyles présentes dans le corps gras.

En particulier :

- lorsque le corps gras est l'huile de ricin, le taux de greffage du corps gras peut être compris entre 5 et 60%, par exemple entre 5 et 40%, par exemple entre 10 et 30%, notamment entre 10 et 15% ;
- lorsque le corps gras est l'huile de colza, le taux de greffage du corps gras peut être compris entre 5 et 80%, par exemple entre 10 et 60 %, par exemple entre 15 et 50% ;
- lorsque le corps gras est l'acide oléique, le taux de greffage du corps gras peut être compris entre 5 et 80%, par exemple entre 10 et 70 %, par exemple entre 15 et 60 %.

Les nombres d'insaturations et de fonctions hydroxyles sont généralement connus et fonctions du corps gras considéré, ils peuvent également être calculés par RMN. Concernant le nombre d'insaturations polymérisées et le nombre de fonctions hydroxyles substituées, ils peuvent être également calculés par RMN.

En particulier, ces calculs peuvent être effectués par RMN du proton, en utilisant du chloroforme deutéré $CDCl_3$ comme solvant.

Le copolymère de l'invention présente avantageusement une masse moléculaire comprise entre 10 000 et $1.10^6$ g/mol, de préférence comprise entre 15 000 et 500 000 g/mol, plus préférentiellement comprise entre 20 000 et 250 000 g/mol, par exemple inférieure à 200 000 g/mol, par exemple inférieure à 150 000 g/mol, par exemple inférieure à 100 000 g/mol, par exemple comprise entre 15 000 et 80 000 g/mol, par exemple comprise entre 20 000 et 80 000 g/mol, par exemple comprise entre 25 000 et 80 000 g/mol.

Ces masses moléculaires peuvent être calculées notamment par chromatographie en phase gazeuse (GPC) avec calibration polystyrène, avec du THF à chaud.

Selon un mode préféré de réalisation, l'invention concerne un copolymère dont le squelette est obtenu par polymérisation radicalaire :

- d'un corps gras (A) comprenant des insaturations choisi parmi les huiles végétales ou d'origine végétales telles que décrites précédemment, en particulier l'huile de ricin ;
- d'au moins un monomère (B) comprenant au moins une fonction polymérisable par polymérisation radicalaire et comprenant au moins une chaine alkyle, linéaire ou ramifiée, comprenant de 16 à 44 atomes de carbone, en particulier l'acrylate de béhényle ;

avec le taux de greffage dudit corps gras étant compris entre 10 et 30%, notamment entre 10 et 15% ; et
avec la masse moléculaire dudit copolymère étant inférieure à 150 000g/mol, par exemple comprise entre 25 000 et 80 00 g/mol.

De façon surprenante, il a été montré par les inventeurs qu'il était possible, grâce au copolymère selon l'invention, de modifier les propriétés, notamment la rhéologie d'un milieu non aqueux avec des polymères de masse moléculaire bien plus faible que celle des polymères usuellement utilisés pour modifier les propriétés, notamment la rhéologie de tels milieux non aqueux.

Selon un mode de réalisation, le copolymère de l'invention peut être obtenu par polymérisation radicalaire d'un corps gras (A), d'au moins un monomère (B) et éventuellement d'au moins un monomère (C) tels que définis précédemment, dans des proportions telles que :

- la quantité molaire dudit monomère (B) par rapport audit monomère (C) varie de 10 à 100%, par exemple de 25 à 100%,
- la quantité molaire dudit monomère (B) par rapport audit corps gras (A) varie de 1 à 99%, par exemple de 10 à 99%,
- la quantité molaire du mélange dudit monomère (B) et dudit monomère (C) par rapport audit corps gras (A) varie de 1 à 99%, par exemple de 10 à 99%.

Le copolymère selon l'invention peut se trouver sous forme liquide ou sous forme solide.

Le copolymère selon l'invention présente des propriétés, notamment des propriétés rhéologiques modifiées, par rapport au corps gras (A), notamment présence d'un seuil rhéologique et/ou modification de la viscosité, et/ou modifications des propriétés de gélification.
De façon avantageuse, le copolymère de l'invention présente un seuil rhéologique identifiable sur la courbe représentant la vitesse de cisaillement en fonction de la contrainte appliquée. Ainsi, la valeur de la vitesse de cisaillement demeure nulle (ou sensiblement nulle) jusqu'à application d'une contrainte minimale, désignée par "valeur seuil de la contrainte".
Sans vouloir être lié à une théorie particulière, les travaux réalisés par les inventeurs dans le cadre de l'invention permettent d'avancer que c'est au moins en partie ce comportement rhéologique particulier qui permettra de maintenir en suspension des composés, par exemple des composés actifs solides ou liquides, par exemple des actifs phytosanitaires, dans des dispersions mettant en oeuvre le copolymère de l'invention.
Dans le cadre de l'invention, la valeur seuil de la contrainte, en deçà de laquelle la vitesse de cisaillement reste nul ou sensiblement nul, est généralement d'au moins 0,01 Pa, de préférence d'au moins 0,1 Pa, par exemple d'au moins 0,5 Pa et ce dans une large gamme de température, notamment aussi bien à température ambiante, notamment de 10 à 30°C, qu'à des températures plus élevées par exemple entre 35 et 60°C, notamment à 45 et à 54°C, et également dans le temps, notamment aussi bien pendant au moins 3 jours, de préférence pendant au moins 7 jours, par exemple pendant au moins 15 jours.
Plus particulièrement, dans le cadre de l'invention, la valeur seuil de la contrainte, en deçà de laquelle la vitesse de cisaillement reste nul ou sensiblement nul, peut être d'au moins 0,01 Pa, de préférence d'au moins 0,1 Pa, par exemple d'au moins 0,5 Pa dans des conditions de vieillissement accéléré standard, par exemple au moins 15 jours à 54°C ou au moins 8 semaines à 45°C.
La détermination des seuils peut être réalisée à partir de tests de fluage sur un rhéomètre AR2000ex (TA Instruments) notamment à 25 et à 54°C. Une géométrie cône-plan est utilisée avec un cône en aluminium d'angle 1deg 59min 2sec, de 60 mm de diamètre et de troncature 57 $\mu$m.
Un pré-cisaillement de 10 $s^{-1}$ pendant 10 s est effectué et l'échantillon est alors laissé au repos pendant 2 min. Des contraintes successives de 1,4 ; 1,6 ; 1,8 ; 2 ; 2,3 ; 2,5 ; 2,8 et 3 Pa à 25°C et de 0,1 ; 0,2 ; 0,3 ; 0,4 ; 0,5 ; 0,6 ; 0,7 ; 0,8 ; 1 ; 1,2 et 1,5 Pa à 54°C sont chacune appliquées pendant 2 min. Pendant ce temps, la déformation du fluide qui en résulte est mesurée. Des tests de recouvrance de 6 min sont réalisés à l'issue de chaque contrainte appliquée. Les résultats obtenus permettent de tracer une courbe de la contrainte en fonction de la vitesse de cisaillement. la vitesse de cisaillement pour chaque contrainte appliquée est alors évalué en prenant la pente de la droite entre 80 et 120 s.
La contrainte seuil, correspond à l'intersection des deux droites caractéristiques de la rupture de pente.

De façon avantageuse, le copolymère de l'invention a une viscosité modifiée, notamment une viscosité plus importante, par rapport au corps gras (A).

De façon avantageuse, le copolymère de l'invention présente des propriétés de gélification et/ou de viscosité modifiées par rapport au corps gras (A), et notamment une viscosité plus importante et/ou une meilleure aptitude à la

gélification.

L'invention concerne également un procédé (P1) de préparation d'un copolymère par polymérisation radicalaire d'un corps gras (A) tel que défini en revendication 1 avec au moins un monomère (B) comprenant au moins une fonction polymérisable par polymérisation radicalaire et comprenant soit au moins une chaîne alkyle, linéaire ou ramifiée, avec ladite chaine comprenant de 16 à 44 atomes de carbone, plus préférentiellement au moins 18 atomes de carbones, par exemple au moins 20 atomes de carbone, notamment 22 atomes de carbone ou 44 atomes de carbone.

Pour le procédé (P1) selon l'invention, les monomères (B) et le corps gras (A) sont tels que définis pour le copolymère selon l'invention.

En outre, selon l'invention, au moins un monomère (C) choisi parmi les monomères $C_N$ neutres ; les monomères $C_A$ anioniques ou potentiellement anioniques ; les monomères $C_C$ cationiques ou potentiellement cationiques ; les monomères $C_z$ zwittérioniques ; les monomères Cp hydrophobes ; leurs mélanges ou association, peut être mis en oeuvre pour la préparation du copolymère.

Les monomères (C) sont tels que définis pour le copolymère selon l'invention.

Selon l'invention, le procédé (P1) est avantageusement mis en oeuvre à une température comprise entre 50 et 150°C, de préférence entre 60 et 120°C.

Selon l'invention, la polymérisation radicalaire est mise en oeuvre en présence d'un initiateur de radicaux qui peut être choisi parmi les radicaux connus de l'homme du métier. A titre d'exemple, on peut citer les peroxydes et les azoïques, de préférence le peroxyde de lauryle et le tertiobutylperoxybenzoate. Il peut notamment être utilisé en une quantité molaire allant de 0,1 à 50%, par exemple de 1 à 20% par rapport au monomère (B).

De préférence, pour le procédé (P1) de l'invention, le ratio molaire B/A correspondant au ratio molaire monomère (B)/corps gras (A) est compris entre 0,1/9,9 et 9,9/0,1, de préférence entre 2/8 et 9/1.

De préférence, pour le procédé (P1) de l'invention, la quantité molaire du monomère (C) par rapport au monomère (B) varie de 0 à 90%, de préférence de 0 à 75%.

La mise en oeuvre du procédé (P1) selon l'invention permet de modifier les propriétés, notamment les propriétés rhéologiques, du corps gras (A). Il permet avantageusement d'apporter un seuil rhéologique et/ou de modifier la viscosité (notamment d'augmenter la viscosité) et/ou de modifier les propriétés de gélification.

La présente invention concerne également un procédé (P2) de modification des propriétés, notamment des propriétés rhéologiques, d'un milieu non aqueux par addition dans ce milieu d'au moins un copolymère selon l'invention.

Le milieu non aqueux selon l'invention est notamment utilisable pour la préparation de compositions dans les domaines de la cosmétique, de l'agrochimie, de la pharmacie, de l'industrie pétrolière, de l'industrie automobile, dans le domaine des encres, des revêtements, etc.

Il doit être compris que le milieu non aqueux selon l'invention peut représenter par exemple tout ou partie de la phase huileuse d'une émulsion, notamment d'une émulsion inverse ou multiple.

Le milieu non aqueux est notamment utilisable dans les formulations agrochimiques par exemple pour réaliser des dispersions en huile (ou « Oil Dispersions » en langue anglaise, également abrégé en « OD »), les émulsions inverses ; dans les solvants tels que les diesters et leurs dérivés, le DMSO, les alcools et leurs dérivés, les éthers et leurs dérivés) ; les compositions issues de l'industrie pétrolière tel que dans le domaine des huiles pétrolières aromatiques, les fluides de forage à base d'huile ; dans les compositions cosmétiques par exemple dans les émulsions inverses, les crèmes, les rouges à lèvres, les déodorants, les vernis ; dans les compositions industrielles telles que les compositions de lubrification, les compositions de revêtements, les peintures, les compositions de décapage, les encres, les graisses ; dans les compositions pharmaceutiques notamment dans les supports de libération de médicament, dans les compositions d'actifs en suspension dans une huile, etc.

De préférence, le milieu non-aqueux est utilisable pour préparer des compositions agrochimiques telles que les dispersions huileuses ou les émulsions inverses ou multiples.

Selon l'invention, le milieu non aqueux peut en outre comprendre des agents complémentaires notamment choisis parmi :

- des agents émulsifiants ;
- des agents épaississants ;
- des agents actifs, notamment agents actifs phytosanitaires, cosmétiques, pharmaceutiques, de préférence des agents phytosanitaires ;
- des polymères hydrosolubles ;
- des polysaccharides, notamment des gommes de guars, xanthane, etc. ; ou
- des agents de formulation.

L'addition du copolymère selon l'invention au milieu non aqueux permet de modifier les propriétés, notamment rhéologiques, de celui-ci, notamment d'apporter un seuil rhéologique et/ou de modifier sa viscosité (notamment augmenter sa viscosité) et/ou de modifier ses propriétés de gélification.

L'ajout du copolymère selon l'invention permet notamment l'apport d'un seuil rhéologique qui permettra de préparer des compositions stables comportant des particules solides en suspension vis-à-vis de la sédimentation des particules solides.

Dans le cadre de l'invention, on entend par « composition stable comportant des particules solides en suspension » une composition qui ne présente sensiblement pas de sédimentation et/ou de séparation de phase du composé dispersé et ce dans une large gamme de température, notamment aussi bien à température ambiante, notamment entre 10 et 30°C, qu'à des températures plus élevées par exemple entre 35 et 60°C, notamment à 45 et 54°C, notamment au moins 16 jours à température ambiante et au moins 15 jours à 54°C. Plus particulièrement, dans le cadre de l'invention, une « composition stable comportant des particules solides en suspension » est une composition qui ne présente sensiblement pas de sédimentation et/ou de séparation de phase du composé dispersé dans des conditions de vieillissement accéléré standard, par exemple au moins 15 jours à 54°C ou au moins 8 semaines à 45°C.

L'ajout du copolymère selon l'invention peut également permettre de modifier la viscosité du milieu non aqueux de départ (notamment d'augmenter sa viscosité), notamment pour limiter les éclaboussures ou l'écoulement lors de l'utilisation des compositions, notamment des produits cosmétiques.

L'ajout du copolymère selon l'invention peut également permettre de modifier les propriétés de gélification du milieu non aqueux, ce qui peut notamment s'avérer utile pour la préparation de compositions qui soient solides mais faciles à étaler, par exemple dans le cadre de composition cosmétique tel que les sticks.

Selon les propriétés rhéologiques qu'il souhaite obtenir, l'homme du métier est à même de déterminer la quantité de copolymère à ajouter au milieu non aqueux.

De préférence dans le procédé (P2), c'est-à-dire lorsque le copolymère est présent à titre d'additif dans le milieu non aqueux, le copolymère est ajouté dans une proportion comprise entre 0,1 et 20 % en poids, par exemple entre 0,5 et 15% en poids, de préférence entre 1 et 7% en poids par rapport au poids total du milieu non aqueux.

Selon une autre variante de réalisation, le copolymère selon l'invention peut être présent à titre principal dans le milieu non aqueux dont on entend modifier les propriétés, notamment rhéologiques, et en particulier le copolymère peut représenter plus de 40% en poids, par exemple plus de 50% en poids, par exemple plus de 60% en poids, par exemple plus de 70% en poids, par exemple plus de 80% en poids, par exemple plus 90% en poids, voire représenter 100% en poids dudit milieu non aqueux.

La présente invention concerne également une composition constituée en tout ou en partie du copolymère selon l'invention.

Ainsi, l'invention concerne une composition totalement constituée d'un copolymère selon l'invention. Dans ce cas la composition pourra être utilisée en tant qu'additif à ajouter dans un milieu non aqueux pour en modifier les propriétés, notamment ses propriétés rhéologiques. Il n'est pas exclu qu'une fraction minoritaire de la composition soit constituée d'autres substances, notamment des agents actifs, des agents d'émulsification, des agents de formulation, des polymères hydrosolubles, des polysaccharides, notamment des gommes de guars, xanthane, à l'exception des milieux non aqueux, sans que ces autres substances ne modifient, ni ne perturbent les propriétés, notamment rhéologiques de cette composition.

L'invention concerne également une composition comprenant au moins un copolymère selon l'invention en mélange avec un milieu non aqueux. Une telle composition présente des propriétés rhéologiques modifiées par rapport à celles du milieu non aqueux permettant ainsi l'utilisation de la composition en tant que base de formulation notamment pour la préparation de compositions dans les domaines de la cosmétique, de l'agrochimie, de la pharmacie, de l'industrie pétrolière, de l'industrie automobile, dans le domaine des encres, des revêtements, etc. Notamment pour la préparation de composition agrochimiques par exemple pour réaliser des dispersions en huile (Oil Dispersion), des émulsions inverses ; dans les solvants tels que les diesters et leurs dérivés, le DMSO, les alcools et leurs dérivés, les éthers et leurs dérivés ; les compositions issues de l'industrie pétrolière tel que dans le domaine des huiles pétrolières aromatiques, les fluides de forage à base d'huile ; dans les compositions cosmétiques par exemple dans les émulsions inverses, les crèmes, les rouges à lèvres, les déodorants, les vernis ; dans les compositions industrielles telles que les compositions de lubrification, les compositions de revêtements, les peintures, les compositions de décapage, les encres, les graisses ; dans les compositions pharmaceutiques notamment dans les supports de libération de médicament, dans les compositions d'actifs en suspension dans une huile, etc.

Les propriétés rhéologiques du milieu non aqueux modifiées par le copolymère vont permettre de préparer des dispersions de composés solides dans le temps et dans une large gamme de température. Notamment, la dispersion pourra être stable après un stockage de 14 jours à 54°C et même après un stockage de 8 semaines à 45°C.

La composition peut également comprendre d'autres substances telles que notamment :

- des agents actifs, par exemple agents actifs phytosanitaires, pharmaceutiques cosmétiques, de préférence des agents actifs phytosanitaires ;
- des agents émulsifiant ;
- des agents épaississant ;

- des polymères hydrosolubles ;
- des polysaccharides, notamment des gommes de guars, xanthane etc. ; et/ou
- des agents de formulation.

De façon plus particulière, la présente invention concerne des compositions émulsifiables par mélange avec de l'eau, comprenant :

- un milieu apolaire ;
- un composé, par exemple un actif phytosanitaire, dispersé au sein dudit milieu apolaire;
- un copolymère selon l'invention; et
- un agent émulsifiant.

Dans le cadre de la présente invention, on entend par « compositions émulsifiables » par mélange avec de l'eau, des compositions qui après mélange avec de l'eau permettent l'obtention d'émulsions.

La composition émulsifiable contient de préférence peu ou pas d'eau. Ainsi, la composition émulsifiable de l'invention comprend de 0 à 5% d'eau, de préférence de 0 à 1% d'eau, par exemple de 0 à 0,1% d'eau, en poids par rapport au poids total de la composition.

La composition émulsifiable selon l'invention peut être tout type de composition émulsifiable qui, notamment selon la nature du composé dispersé, peut avoir différentes utilisations. La composition émulsifiable selon l'invention peut par exemple être une composition cosmétique, une composition agrochimique, une composition pharmaceutique, une composition utilisable dans les industries du pétrole, de l'automobile, des encres, des revêtements, etc. De façon plus particulière, la composition émulsifiable selon l'invention est une composition agrochimique, notamment une dispersion huileuse (ou *oil dispersion* « OD » en anglais).

Dans le cadre de l'invention, un composé est dit dispersé au sein d'un milieu apolaire lorsque moins de 5% en poids, de préférence moins de 3% en poids, par exemple moins de 1% en poids, de ce composé est sous forme solubilisée dans ledit milieu apolaire, notamment à température ambiante, généralement de 15 à 30°C, voire à une température allant jusqu'à 54°C.

Le composé dispersé peut être un composé solide dispersé ou un composé liquide dispersé.

Selon un mode de réalisation de l'invention, le composé dispersé est un composé solide dispersé. Dans le cadre de l'invention on entend par composé solide un composé dont le point de fusion est supérieur ou égal à 50°C.

Selon un autre mode de réalisation de l'invention, le composé dispersé est un composé liquide dispersé. Dans le cadre de l'invention on entend par composé liquide un composé dont le point de fusion est inférieur à 50°C.

Dans le cadre de la présente invention, les inventeurs ont maintenant mis en évidence que les compositions comprenant les composés précités s'avèrent stables, notamment à température ambiante et à des températures élevées, notamment à 45°C, voire jusqu'à 54°C. Les compositions selon l'invention s'avèrent également stables dans le temps, notamment au moins 16 jours à température ambiante et au moins 15 jours à 54°C. En particulier, on n'observe avantageusement pas de sédimentation et/ou de séparation de phase à ces températures et dans ces durées.
Plus concrètement, les travaux qui ont été réalisés par les inventeurs ont maintenant permis de mettre en évidence que l'association du milieu apolaire et du copolymère selon l'invention permet d'induire un comportement viscoélastique de ce milieu dispersant apolaire qui s'avère suffisant pour permettre un maintien en suspension du composé, et l'écoulement de la formulation, et ce aussi bien à température ambiante, notamment entre 10 et 30°C, notamment au moins 16 jours à température ambiante, qu'à température plus élevée, par exemple entre 35 et 60°C, notamment à 45 et 54°C, notamment dans des conditions de vieillissement accéléré standard (par exemple au moins 15 jours à 54°C ou au moins 8 semaines à 45°C), et ce en dépit de la présence de l'agent émulsifiant.

La mise en oeuvre de l'association du milieu apolaire et du copolymère selon l'invention permet avantageusement d'induire un seuil rhéologique dans la courbe représentant le cisaillement en fonction de la contrainte appliquée. Ainsi, la valeur du cisaillement demeure nulle (ou sensiblement nulle) jusqu'à application d'une contrainte minimale, désignée par "valeur seuil de la contrainte". Sans vouloir être lié à une théorie particulière, les travaux réalisés par les inventeurs dans le cadre de l'invention permettent d'avancer que c'est au moins en partie ce comportement rhéologique particulier qui permet de maintenir en suspension le composé, notamment les actifs phytosanitaires, dans les compositions de l'invention. Dans le cadre de l'invention, la valeur seuil de la contrainte, en deçà de laquelle le cisaillement reste nul ou sensiblement nul, est généralement supérieure à 0,2 Pa, de préférence comprise entre 0,2 et 1 Pa et ce dans une large gamme de température, notamment aussi bien à température ambiante, notamment entre 10 et 30°C, qu'à des températures plus élevées par exemple entre 35 et 60°C, notamment à 45 et 54°C, notamment au moins 16 jours à température ambiante, et notamment dans des conditions de vieillissement accéléré standard (par exemple au moins 15 jours à 54°C ou au moins 8 semaines à 45°C). De façon surprenante, il s'avère que ce comportement rhéologique surprenant est obtenu en présence de l'agent émulsifiant et sur une large gamme de température. La présence de l'agent émulsifiant permet de réaliser une émulsion relativement stable à partir de la composition en la mélangeant à

de l'eau et sur une large gamme de température. Ainsi, les compositions de l'invention se révèlent particulièrement adaptées à titre d'OD, permettant un maintien en suspension de composés, notamment d'actifs phytosanitaires, au sein d'un milieu apolaire, notamment d'une huile, ce qui autorise leur stockage et transport sous forme concentrée et leur conversion aisée en émulsion par simple mélange avec de l'eau sur le site d'utilisation.

Dans le cadre de l'invention, on entend par composition stable une composition qui ne présente sensiblement pas de sédimentation et/ou de séparation de phase du composé dispersé et ce dans une large gamme de température, notamment aussi bien à température ambiante, notamment entre 10 et 30°C, qu'à des températures plus élevées par exemple entre 35 et 60°C, notamment à 45 et 54°C, notamment au moins 16 jours à température ambiante et au moins 15 jours à 54°C. De préférence, une composition stable est une composition qui ne présente sensiblement pas de sédimentation et de séparation de phase du composé dispersé et ce dans une large gamme de température, notamment aussi bien à température ambiante, notamment entre 10 et 30°C, qu'à des températures plus élevées par exemple entre 35 et 60°C, notamment à 45 et 54°C, notamment au moins 16 jours à température ambiante et en particulier dans des conditions de vieillissement accéléré standard (par exemple au moins 15 jours à 54°C ou au moins 8 semaines à 45°C).

De préférence, une composition selon l'invention reste stable après un stockage de 15 jours à 54°C. Egalement, une composition selon l'invention peut rester stable après un stockage de 8 semaines à 45°C.

**Composé dispersé**

Le composé, notamment actif phytosanitaire, présent à l'état dispersé au sein de la composition selon l'invention peut être présent en une concentration élevée, pouvant dans certains cas aller jusqu'à 30%, par exemple 40 %, voire 50 %, en poids par rapport au poids total de la composition, sans perte de la stabilité de la dispersion.
Le plus souvent toutefois, la concentration en composé présent à l'état dispersé (généralement actif phytosanitaire dispersé) est de 0,1 à 20% en poids, par exemple de 0,5 à 10 % en poids, par exemple de 1 à 8 % en poids par rapport au poids total de la composition.

Le composé présent à l'état dispersé au sein de la composition selon l'invention peut être choisi parmi tout composé non soluble dans le milieu apolaire de cette composition. Dans le cadre de l'invention, un composé est dit non soluble lorsque moins de 5%, de préférence moins de 3%, par exemple moins de 1% de ce composé est sous forme solubilisée dans le milieu apolaire. Il peut typiquement s'agir d'un actif phytosanitaire, à savoir un actif propre à améliorer la croissance des végétaux, à soigner ou prévenir les maladies des végétaux; ou à lutter contre des parasites ou des espèces nuisibles propres à inhiber ou modifier la croissance de la plante. Un actif phytosanitaire peut par exemple être un pesticide, tel qu'un insecticide, un bactéricide, un fongicide, un herbicide, ou un régulateur de croissance des plantes ou des organismes nuisibles, ou leurs mélanges.

Comme actif fongicide, on peut citer les inhibiteurs de la synthèse d'acide nucléique ; les inhibiteurs de la mitose et de la division cellulaire ; les inhibiteurs de la respiration ; les composés capables d'agir comme découpleur ; les inhibiteurs de la synthèse d'ATP production ; les inhibiteurs de la biosynthèse des acides aminés ou des protéines ; les inhibiteurs de la transduction du signal ; les inhibiteurs de la synthèse lipidique au niveau de la membrane ; les inhibiteurs de la biosynthèse de l'ergosterol ; les inhibiteurs de la synthèse de la paroi cellulaire ; les inhibiteurs de la biosynthèse de la mélanine ; les composés capables de déclencher des mécanismes de défense des plantes ; les composés capables d'agir en plusieurs sites ; ou leurs mélanges.

Comme matière active insecticide, on peut citer les inhibiteurs de l'acetylcholinesterase (AChE) ; les composés antagonistes de la voie chlorure-GABA-dépendante ; les composés antagonistes des récepteurs nicotino-acetylcholine ; les composés modulateurs des récepteurs allosteric-acetylcholine ; les composés activateurs de la voie chlorure ; les composés dont le mode d'action n'est pas connu ou n'est pas spécifique, par exemple agents gazant ; ou inhibiteurs de la croissance des mites ; les inhibiteurs de la phosphorylation oxydante ; les perturbateurs de l'ATP ; les composés découpleurs de la phoshorylation oxydante ; les perturbateurs microbiens de la membrane digestive des insectes ; les inhibiteurs de la biosynthèse de la chitine ; les perturbateurs de la mue ; les perturbateurs de l'ecdysone ; les anti-octopaminergic ; les inhibiteurs du transport des électrons (site II ou site III) ; les inhibiteurs du transport des électrons (site I) ; les inhibiteurs de la biosynthèse des acides gras ; les inhibiteurs neuronaux à mode d'action non élucidé ; les composés capables de modifier les récepteurs Ryanodine ; ou leurs mélanges.

A titre préféré d'actifs phytosanitaires utilisables dans les compositions émulsifiables, on peut notamment citer, à titre non limitatif, les sulfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl, les azoles tels que le difenconazole, la famille des fongicide triazoles tels que l'azaconazole, bromuconazole, cyproconazole, difenoconazole, diniconazole, epoxyconazole, fenbuconazole, flusilazole, myclobutanyl, tebuconazole, ou bien encore l'amétryne, le diuron, le linuron, le novaluron, le chlortoluron, l'isoproturon, le metamitron, le diazinon, l'aclonifen, l'atrazine, le chlorothalonil, le bromoxynil, le bromoxynil heptanoate, le bromoxynil octanoate, le mancozeb, la manèbe, le zineb, la phenmédipham, le propanyl, la série des phénoxyphénoxy, la série des hétéroaryloxyphénoxy, le CMPP, le MCPA, le 2,4-D, la simazine, les produits actifs de la série des imidazolinones, la famille des organophosphorés, avec notamment l'azinphos-éthyl, l'azinphos-méthyl, l'alachlore, le chlorpyriphos, le diclofop-méthyl, le fénoxaprop-p-éthyl, le méthoxychlore, la cyperméthrine, l'alpha-cyperméthrine, le phenmedipham, le propanil, l'oxyfluorfen, le dimethoate, l'imidacloprid, le propoxur, le benomyl, la deltamethrine, le fenvalerate, l'abamectin, l'amicarbazone, le bifenthrin, le carbosulfan, le cyfluthrin, l'ethofenprox, le fenoxaprop-ethyl, le fluazifop-p-butyl, le flufenoxuron, l'hexazinone, le lambda-cyalothrin, le permethrin, le prochloraz, le methomyl, le fenoxycarbe, le cymoxanil, le chlorothalonyl, les insecticides neonicotinoides, triadimefon, triadimenol, des strobilurines telles que la pyraclostrobine, la picoxystrobine, l'azoxystrobine, la famoxadone, le kresoxym-methyl et la trifloxystrobine ; ou leurs mélanges.

L'invention se révèle notamment bien adaptée à la mise en oeuvre d'actifs de la famille des sulfonylurées, tel que le nicosulfuron et des azoles tel que le tébuconazole.

La composition selon l'invention peut en outre comprendre, selon un mode de réalisation particulier, un ou plusieurs autres composés actifs (généralement d'autres actifs phytosanitaires ou des composés modulant la phytotoxicité des actifs (phytoprotecteur ou « safener » en anglais)) qui sont solubilisés ou miscibles dans le milieu apolaire. Les compositions de ce type, qui comprennent des actifs en combinaison sous deux formes distinctes sont des formulations bien adaptées notamment en agrochimie, qui sont parfois désignées par le terme de "combos".

Selon un mode de réalisation, le composé dispersé peut être un composé solide dispersé au sein des compositions de l'invention (généralement un actif phytosanitaire ou un mélange d'actifs phytosanitaires à l'état solide). Il se présente de préférence sous la forme d'objets dispersés (particules ou agrégats de particules) ayant des dimensions inférieures à 50 $\mu$m, notamment inférieures à 20 $\mu$m, avantageusement comprises entre 1 et 15 $\mu$m, par exemple de l'ordre de 10 $\mu$m ou moins. La taille de ces objets en suspension peut être déterminée selon tout moyen connu en soi, par exemple par microscopie optique ou diffusion de la lumière.

Selon un autre mode de réalisation, le composé dispersé peut être un composé liquide dispersé au sein des compositions de l'invention (généralement un actif phytosanitaire ou un mélange d'actifs phytosanitaires à l'état liquide). Il se présente de préférence sous la forme de gouttelettes ayant des dimensions inférieures à 5 $\mu$m, notamment comprises entre 0,5 et 5 $\mu$m, par exemple entre 1 et 3 $\mu$m. La taille de ces gouttelettes peut être déterminée selon tout moyen connu en soi, par exemple par microscopie optique ou diffusion de la lumière.

De manière particulièrement préférée, le composé dispersé est le nicosulfuron.

**<u>Milieu apolaire</u>**

Au sens de l'invention, on entend par « milieu apolaire » tout constituant liquide à la température de préparation ou d'utilisation de la composition, qui, situé dans l'espace de solubilité de HANSEN (Handbook of solubility parameters and other cohesion parameters - Allan F.M. BARTON, CRC Press Inc., 1983 -), présente les paramètres suivants :

- $\delta P$ d'interactions Keesom inférieur à 10 $(J/cm^3)^{1/2}$
- $\delta H$ de liaisons hydrogène inférieur à 10 $(J/cm^3)^{1/2}$
- $\delta D$ d'interactions London supérieur à 15 $(J/cm^3)^{1/2}$

A titre d'exemple de milieu apolaire ou milieu de dispersion apolaire on peut citer :

- les triglycérides d'acides gras saturés ou insaturés comprenant au moins 12 atomes de carbone et de préférence de 14 à 22 atomes de carbone ; il peut s'agir de triglycérides de synthèse ou de préférence de triglycérides naturels, tels que les huiles végétales ou d'origine végétales du type huile de colza, huile de soja, huile d'arachide, huile de beurre, huile de graine de coton, huile de lin, huile de noix de coco, huile d'olive, huile de palme, huile de pépin de raisin, huile de ricin, huile de coprah, ou les huiles animales ou d'origine animales, par exemple huiles de poisson, notamment les huiles de poisson comprenant des oméga 3 ;
- les esters des triglycérides d'acides gras saturés ou insaturés comprenant au moins 12 atomes de carbone et de préférence de 14 à 22 atomes de carbone (notamment leurs esters méthyliques et éthyliques) ;
- les coupes pétrolières aromatiques ;
- les solvants aromatiques (anisole, toluène, par exemple) ;
- les composés terpéniques (D-limonène, L-limonène, par exemple) ;
- les mélanges de diesters succinate/adipate/glutarate de diméthyle ;
- les hydrocarbures aliphatiques comprenant au moins 6 atomes de carbone (isooctane, kerosène, essence, essence diesel, huiles minérales (notamment huile de paraffine), huiles lubrifiantes...) ; ou
- leurs mélanges.

De manière avantageuse, le milieu apolaire présent dans la composition de l'invention comprend, voire est, un mélange de triglycérides, par exemple une huile végétale ou d'origine végétale choisie par exemple parmi l'huile de colza, l'huile de soja, l'huile de maïs, l'huile de ricin, l'huile d'arachide, l'huile de beurre, l'huile de graine de coton, l'huile

de lin, l'huile de noix de coco, l'huile d'olive, l'huile de palme, l'huile de pépin de raisin, l'huile de coprah et leurs mélanges. L'huile de colza, notamment, est bien adaptée à l'invention, de même que les huiles de maïs et de soja, et leurs mélanges. De préférence, le milieu apolaire est l'huile de colza.

L'utilisation préférentielle d'huile végétale ou d'origine végétale permet avantageusement de se passer des solvants généralement utilisés, tels que le xylène, le naphtalène, la N-méthylpyrrolidone, la cyclohexanone ou bien encore l'isophorone, dans les concentrats émulsifiables (ou EC, pour l'anglais *"emulsifiable concentrate*") et qui ont un impact non négligeable sur l'environnement.

On peut également utiliser d'autres huiles naturelles telles que par exemple les huiles animales ou d'origine animales, notamment les huiles de poisson, par exemple les huiles de poisson comprenant des oméga 3.

Le milieu apolaire présent dans la composition peut également comprendre, voire être, une coupe pétrolière aromatique telle que les Solvesso (par exemple le SOLVESSO 100 qui est un mélange de dialkyl et trialkyl benzènes en $C_9$ à $C_{10}$, le SOLVESSO 150 qui contient en mélange principalement des alkylbenzènes en $C_{10}$ à $C_{11}$ ou le SOLVESSO 200, qui contient principalement des alkyl naphtalènes en $C_{10}$ à $C_{14}$), de préférence le SOLVESSO 200 ND, de préférence en mélange avec d'autres composés tels que des esters d'huiles végétales ou d'origine végétales, notamment d'huile de colza, du type du Phytorob 926-25 commercialisé par la société Novance ou Amesolve CME disponible auprès de Ametech. Ce mode de réalisation est notamment adapté aux compositions comprenant des composés modulant la phytotoxicité des actifs de type phytoprotecteurs.

Le milieu apolaire peut être de nature identique ou différente du corps gras (A) selon l'invention.
Selon un mode de réalisation, le milieu apolaire et le corps gras (A) peuvent être des huiles végétales ou d'origine végétales. Par exemple, le milieu apolaire peut être l'huile de colza et le corps gras (A) peut être l'huile de ricin.

Généralement, quelle que soit la nature du milieu apolaire, par exemple de l'huile, celui-ci peut être présent dans la composition selon l'invention à une teneur allant de 40 à 99 % en poids, de préférence de 40 à 90%, cette teneur étant de préférence d'au moins 60% en poids, par exemple d'au moins 65% en poids, par exemple comprise entre 65 et 75% en poids, par rapport au poids total de la composition.

Il n'est pas exclu de mélanger à des huiles végétales ou d'origine végétale ou naturelle des coupes aromatiques, dans des proportions variables. L'utilisation de telles coupes aromatiques peut notamment être mise en oeuvre lorsque les compositions comprennent en plus de l'actif dispersé un composé phytoprotecteur (« safener » en anglais), ces coupes pétrolières pouvant permettre alors avantageusement de solubiliser le phytoprotecteur.
Par exemple, le milieu apolaire peut comprendre des huiles végétales ou naturelles et des coupes aromatiques dans des proportions massiques allant de 20:80 à 80:20, de préférence allant de 65:35 à 75:25.

Selon un mode de réalisation le milieu apolaire peut comprendre des huiles végétales ou naturelles et des coupes aromatiques dans des proportions massiques allant de 40:60 à 60:40, et par exemple 50:50.

Il n'est pas exclu non plus de mélanger à des huiles végétales ou d'origine végétale ou naturelle des esters méthylique d'huile végétale, dans des proportions variables.

Quelle que soit la nature du copolymère selon l'invention mis en oeuvre, celui-ci est de préférence employé à une concentration suffisamment faible pour éviter une prise en masse du milieu, mais suffisante néanmoins pour induire la modification rhéologique du milieu recherchée pour assurer la stabilisation des composés à l'état dispersé dans la composition. La quantité de copolymère selon l'invention dans la composition émulsifiable est généralement comprise entre 0,5 et 40% en poids, par exemple entre 1 et 20% en poids, de préférence entre 2 et 10 en poids, par exemple entre 3 et 8% en poids, par rapport au poids total de la composition.

A cet effet, notamment lorsque le milieu apolaire présent dans la composition comprend au moins, par exemple comprend majoritairement, par exemple comprend au moins 50% en poids, voire est une huile à base de triglycérides, comme une huile végétale par exemple, la concentration en copolymère selon l'invention est de préférence inférieure à 10%, plus avantageusement inférieure à 8% en poids par rapport au poids total de la composition, typiquement entre 1 et 8%, par exemple entre 3 et 6%, notamment de l'ordre de 4 à 6 % en poids par rapport au poids total de la composition.

De façon particulièrement préférée, dans les compositions émulsifiables selon l'invention, le milieu apolaire est une huile végétale ou d'origine végétale telle que décrite précédemment.

En particulier, lorsque le copolymère selon l'invention est un copolymère dont le squelette est obtenu par polymérisation radicalaire :

- d'un corps gras (A) comprenant des insaturations choisi parmi les huiles végétales ou d'origine végétales telles que décrites précédemment, en particulier l'huile de ricin ;
- d'au moins un monomère (B) comprenant au moins une fonction polymérisable par polymérisation radicalaire et comprenant au moins une chaine alkyle, linéaire ou ramifiée, comprenant de 16 à 44 atomes de carbone, en particulier l'acrylate de béhényle ;

avec le taux de greffage dudit corps gras étant compris entre 10 et 30%, notamment entre 10 et 15% ; et
avec la masse moléculaire dudit copolymère étant inférieure à 150 000g/mol, par exemple comprise entre 25 000 et 80

00 g/mol,
il peut être avantageusement formulé avec une huile végétale ou d'origine végétale telle que définie précédemment, notamment avec de l'huile de colza.
Ce mode de réalisation se révèle notamment bien adapté à la mise en oeuvre d'actifs de la famille des sulfonylurées, tel que le nicosulfuron et des azoles tel que le tébuconazole.

**Agent émulsifiant**

L'agent émulsifiant, présent dans la composition selon l'invention, est propre à assurer une émulsification du milieu apolaire, par exemple de l'huile, lors d'un mélange de la composition avec de l'eau ou un milieu aqueux; il peut être choisi parmi les agents tensioactifs adaptés à l'émulsification du milieu apolaire, par exemple de l'huile, spécifique présente dans la composition émulsifiable.
Ainsi, par exemple lorsque le milieu apolaire comprend au moins, par exemple comprend majoritairement, par exemple comprend au moins 50 % en poids, voire est, un mélange de triglycérides telle qu'une huile végétale, l'agent émulsifiant peut être choisi parmi les tensioactifs non ioniques de type acides gras ou esters tels que par exemple les esters, ester de glycol, esters de glycerol, esters de PEG, PEG esters d'acides gras, ester de sorbitol, ester de sorbitol ethoxylés, acides ethoxylés, ou ethoxy propoxylés, esters et triglycérides (famille des Alkamuls® de RHODIA), notamment huiles de riçin éthoxylées et leurs mélanges.
L'agent émulsifiant peut notamment être choisi parmi les huiles de riçin éthoxylées, les polyéthylènes glycol esters d'acides gras et les esters de sorbitan, et leurs mélanges.
A titre d'exemple d'huiles de riçin éthoxylées convenant à l'invention, on peut notamment citer les produits Alkamuls® OR 36, Alkamuls® RC, Alkamuls® R81, Alkamuls® 696 et Alkamuls OR/10 disponibles auprès de la Société Rhodia.
A titre d'exemple de polyéthylène glycol esters d'acides gras convenant à l'invention, on peut notamment citer le produit Alkamuls VO/2003, disponible auprès de la Société Rhodia.
A titre d'exemple d'esters de sorbitan convenant à l'invention, on peut notamment citer les produits Alkamuls T/85-V, Alkamuls T/80, disponibles auprès de la Société Rhodia.
Des agents émulsifiants particulièrement bien adaptés, notamment lorsque le milieu apolaire comprend au moins, par exemple comprend majoritairement, par exemple comprend au moins 50 % en poids, voire est, un mélange de triglycérides telle qu'une huile végétale, sont les polyéthylènes glycol esters d'acides gras, seuls ou en association avec un autre agent tensioactif.
Un agent émulsifiant particulièrement bien adapté dans le cadre des compositions émulsifiables de l'invention est l'Alkamuls VO/2003 disponible auprès de la Société Rhodia.
Pour d'autres milieux apolaires, en particulier des milieux apolaires comprenant au moins, par exemple comprenant majoritairement, par exemple comprenant au moins 50 % en poids, voire étant, des coupes pétrolières aromatiques et/ou des hydrocarbures aliphatiques comprenant au moins 6 atomes de carbone, des agents émulsifiants adaptés sont notamment des tensioactifs anioniques tels que les sulfonates, sulfonates aliphatiques, sulfonates portant des groupements ester ou amide tels que les isothionate (sulfoester), taurates (sulfoamides), sulfosuccinates, sulfosuccinamates, ou encore les sulfonates ne portant pas de groupements amide ou ester tels que les alkyldiphenyoxide disulfonate, Alkyl naphtalène sulfonate, naphtalène/formaldéhyde sulfonates avec par exemple les Dodecyl benzene sulfonate (famille Rhodacal® de RHODIA, comme par exemple le Rhodacal® 60 BE), seuls ou en association avec :

- un ou plusieurs tensioactifs non ioniques de type acides gras ou esters tels que par exemple les esters, ester de glycol, esters de glycerol, esters de PEG, ester de sorbitol, ester de sorbitol ethoxylés, acides ethoxylés, ou ethoxy propoxylés, esters et triglycérides (famille des Alkamuls® de Rhodia), notamment huiles de riçin éthoxylées ; de préférence un ou plusieurs tensioactifs non ioniques de type huiles de riçin éthoxylées, comme par exemples celles commercialisées par la Société Rhodia sous les références Alkamuls® OR 36, Alkamuls® RC, Alkamuls® R81, Alkamuls® 696, et/ou
- un ou plusieurs tensioactifs choisis parmi les composés à base de styryl phénol tels que les Distyrylphénol, Tristyrylphénol, qui peuvent-être ethoxylés ou éthoxy propoxylés, phosphatés, sulfatés, par exemple la famille des Soprophor® commercialisée par la Société Rhodia comme le Soprophor® DSS7, le Soprophor® BSU, le Soprophor® 3D33, le Soprophor® 4D384, le Soprophor® 796P.

Sont notamment adaptés les tensioactifs de type Rhodasurf®, Rhodacal® et Alkamuls® disponibles auprès de la Société Rhodia.
Une association de tensioactifs bien adaptée à ces milieux apolaires est celle commercialisée sous le nom de Géronol MOE/02-K par la Société Rhodia.
La teneur en agent émulsifiant peut varier en fonction notamment de la nature du milieu apolaire et de la nature de l'agent émulsifiant employé.

En général, la teneur en agent émulsifiant au sein de la composition émulsifiable selon l'invention est typiquement comprise entre 3 et 30% en poids, par rapport au poids total de la composition émulsifiable.
De préférence, lorsque le milieu apolaire comprend au moins, par exemple comprend majoritairement, par exemple comprend au moins 50 % en poids, voire est, un mélange de triglycérides tel qu'une huile végétale, la teneur en agent émulsifiant est préférentiellement comprise entre 5 et 25% en poids, par exemple entre 10 et 20 % en poids, par exemple d'environ 15% en poids, par rapport au poids total de la composition émulsifiable.
De préférence, lorsque le milieu apolaire comprend au moins, par exemple comprend majoritairement, par exemple comprend au moins 50 % en poids, voire est, un hydrocarbure aliphatique comprenant au moins 6 atomes de carbone, par exemple une huile minérale, la teneur en agent émulsifiant est préférentiellement comprise entre 3 et 15% en poids, par exemple entre 4 et 10 % en poids, par exemple d'environ 5% en poids, par rapport au poids total de la composition émulsifiable.
Dans un mode de réalisation particulier dans la composition émulsifiable selon l'invention le milieu apolaire est une huile végétale ou un mélange d'huiles végétales, telle que décrite ci-dessus, et l'émulsifiant est un polyéthylène glycol esters d'acides gras (notamment de type Alkamuls VO/2003, disponible auprès de la Société Rhodia).
Dans un autre mode de réalisation particulier dans la composition émulsifiable selon l'invention le milieu apolaire est une huile végétale ou un mélange d'huiles végétales, telle que décrite ci-dessus, l'émulsifiant est un polyéthylène glycol esters d'acides gras (notamment de type Alkamuls VO/2003, disponible auprès de la Société Rhodia) et le composé dispersé est le nicosulfuron.
Le milieu apolaire peut être en particulier l'huile de colza et l'émulsifiant un polyéthylène glycol esters d'acides gras (notamment de type Alkamuls VO/2003, disponible auprès de la Société Rhodia).
Ce mode de réalisation est particulièrement adapté lorsque le copolymère selon l'invention est un copolymère dont le squelette est obtenu par polymérisation radicalaire :

- d'un corps gras (A) comprenant des insaturations choisi parmi les huiles végétales ou d'origine végétales telles que décrites précédemment, en particulier l'huile de ricin ;
- d'au moins un monomère (B) comprenant au moins une fonction polymérisable par polymérisation radicalaire et comprenant au moins une chaine alkyle, linéaire ou ramifiée, comprenant de 16 à 44 atomes de carbone, en particulier l'acrylate de béhényle ;

avec le taux de greffage dudit corps gras étant compris entre 10 et 30%, notamment entre 10 et 15% ; et
avec la masse moléculaire dudit copolymère étant inférieure à 150 000g/mol, par exemple comprise entre 25 000 et 80 00 g/mol.

**Ingrédients optionnels**

La composition émulsifiable selon l'invention peut également comprendre éventuellement d'autres ingrédients, notamment choisis parmi :

- des charges, par exemple des particules de silice, à l'image par exemple de la silice commercialisée par la société Evonik sous la référence Aerosil® 200 ;
- des sels, en particulier des carbonates ou des sulfates, comme par exemple le carbonate de sodium ou le sulfonate d'ammonium (l'ajout de tels sels peut être notamment conseillé lors de l'utilisation d'eau dure) ;
- des agents anti-mousse, à l'image par exemple de celui commercialisé par la société Rhodia sous la référence Silcolapse RG22 ;
- et leurs mélanges.

En général, la teneur en charges (si présentes), par exemple des particules de silice, est inférieure à 1% en poids, de préférence inférieure à 0,5% en poids, de préférence inférieure à 0,2% en poids, par exemple comprise entre 0,05 et 0,1% en poids, par rapport au poids total de la composition émulsifiable selon l'invention.
En général, la teneur en sels (si présents), par exemple en carbonate de sodium ou en sulfonate d'ammonium, est inférieure à 1% en poids, de préférence inférieure à 0,5% en poids, de préférence inférieure à 0,2% en poids, par exemple comprise entre 0,05 et 0,1% en poids, par rapport au poids total de la composition émulsifiable selon l'invention.
En général, la teneur en agent anti-mousse (si présent) est inférieure à 1% en poids, de préférence inférieure à 0,5% en poids, de préférence inférieure à 0,2% en poids, par exemple comprise entre 0,05 et 0,1% en poids, par rapport au poids total de la composition émulsifiable selon l'invention.
Selon sa composition, la composition émulsifiable selon l'invention peut typiquement être préparée en mettant en oeuvre un procédé (P1) comprenant les étapes suivantes :

(i) on mélange le milieu apolaire et l'agent émulsifiant (ces composés étant de préférence introduits dans cet ordre pour former le mélange), ledit mélange est avantageusement cisaillé, notamment au moyen d'un dispositif de type pâle défloculeuse ; puis

(ii) on ajoute le copolymère selon l'invention, le milieu obtenu est avantageusement cisaillé, notamment au moyen d'un dispositif de type pâle défloculeuse ; on chauffe (si nécessaire) jusqu'à solubilisation du copolymère (généralement entre 60 °C et 75 °C) avant de refroidir le tout sous agitation ; puis

(iii) on ajoute le composé à disperser, le milieu obtenu est avantageusement cisaillé, notamment au moyen d'un dispositif de type Ultraturax.

Selon un mode de réalisation, à l'issue de l'étape (iii), la composition peut être soumise à un broyage, notamment dans un broyeur humide.
Une telle étape additionnelle de broyage peut notamment être avantageuse pour réduire la taille des particules lorsque le composé à disperser est un composé solide de taille importante, afin d'obtenir par exemple une taille des particules ou agrégats en dispersion de préférence inférieure à 20 $\mu$m, plus avantageusement inférieure à 10 $\mu$m.
Une étape additionnelle de broyage peut également être avantageuse, pour d'autres raisons, lorsque le composé à disperser est un composé liquide ou un composé solide de taille convenable. Elle peut notamment permettre d'améliorer le mélange des ingrédients.

Les compositions émulsifiables selon l'invention peuvent également être obtenues selon un procédé (P2), comprenant les étapes suivantes :

(a) on mélange le milieu apolaire, l'agent émulsifiant et le composé à disperser (ces composés étant de préférence introduits dans cet ordre pour former le mélange), ledit mélange est avantageusement cisaillé, notamment au moyen d'un dispositif de type Ultraturax ; puis

(b) on ajoute à chaud le copolymère selon l'invention solubilisé dans du milieu apolaire (généralement entre 60°C et 75°C).

Selon un mode de réalisation, l'étape (b) est précédée d'une étape (b1) de broyage du mélange obtenu à l'étape (a), notamment dans un broyeur humide, de préférence dans le cas où le composé à disperser est un composé solide jusqu'à obtention d'une taille de particules ou d'agrégats en dispersion inférieure à 20 $\mu$m, plus avantageusement inférieure à 10 $\mu$m. Dans ce mode de réalisation l'étape (b) est également mise en oeuvre au cours du broyage.
Une étape additionnelle de broyage peut également être avantageuse, pour d'autres raisons, lorsque le composé à disperser est un composé liquide ou un composé solide de taille convenable. Elle peut notamment permettre d'améliorer le mélange des ingrédients.

Les compositions émulsifiables selon l'invention peuvent également être obtenues selon un procédé (P3), comprenant les étapes suivantes :

(I) on mélange le milieu apolaire et le composé à disperser (ces composés étant de préférence introduits dans cet ordre pour former le mélange), ledit mélange est avantageusement cisaillé, notamment au moyen d'un dispositif de type Ultraturax ; puis

(II) on ajoute à chaud le copolymère selon l'invention solubilisé dans du milieu apolaire et l'agent émulsifiant (généralement entre 60°C et 75°C).

Selon un mode de réalisation, l'étape (II) est précédée d'une étape (II1) de broyage du mélange obtenu à l'étape (I), notamment dans un broyeur humide, de préférence dans le cas où le composé à disperser est un composé solide jusqu'à obtention d'une taille de particules ou d'agrégats en dispersion inférieure à 20 $\mu$m, plus avantageusement inférieure à 10 $\mu$m. Dans ce mode de réalisation l'étape (II) est également mise en oeuvre au cours du broyage. Une étape additionnelle de broyage peut également être avantageuse, pour d'autres raisons, lorsque le composé à disperser est un composé liquide ou un composé solide de taille convenable. Elle peut notamment permettre d'améliorer le mélange des ingrédients.

La composition selon l'invention est notamment utilisable pour la préparation sur site d'émulsions pour la délivrance de composés, notamment solides, notamment d'actifs phytosanitaires, par dilution dans de l'eau. L'émulsion obtenue, peut par exemple être employée pour la pulvérisation sur les parties aériennes, notamment le feuillage, de plantes.

Dans ce cadre, la composition selon l'invention peut être utilisée pour former des émulsions de type huile-dans-l'eau comprenant le composé à l'état dispersé. La formation d'une telle émulsion est généralement réalisée par mélange d'une composition émulsifiable (de type OD) selon l'invention avec une phase aqueuse, notamment avec de l'eau. De préférence, le rapport massique composition selon l'invention/eau est compris entre 0,1 : 100 et 10 : 100, plus préférentiellement entre 1 entre 1 : 100 et 5 : 100, par exemple entre 2 :100 et 3 :100.

Les émulsions obtenues à partir des compositions selon l'invention permettent notamment une bonne dispersion des actifs à la surface des plantes à traiter. De plus, la mise en oeuvre de l'actif au sein d'un véhicule apolaire permet généralement une bonne comptabilisation de l'actif avec la surface à traiter, ce qui peut notamment permettre une persistance de l'activité sur la surface de la plante, une meilleure pénétrabilité et une bonne tenue de l'actif, y compris lors d'intempéries (vent et pluie notamment). Les émulsions obtenues présentent notamment une meilleure biodisponibilité et une résistance accrue au lessivage.

Dans le cas où le copolymère selon l'invention présenterait une viscosité élevée, les inventeurs ont par ailleurs découvert que l'ajout d'un émulsifiant liquide particulier pouvait, de façon particulièrement avantageuse, conduire à un mélange concentré bi-fonctionnel (agent rhéologique et émulsifiant) homogène, liquide, coulable et donc plus facilement manipulable. Ce mélange bi-fonctionnel est particulièrement avantageux dans le cas notamment des formulations OD, qui requièrent la présence d'un tel émulsifiant dans la formulation finale. Ainsi, selon un autre mode de réalisation, la présente invention concerne également des compositions concentrées comprenant un copolymère selon l'invention et un agent émulsifiant. De préférence, la composition concentrée selon l'invention consiste en un mélange d'un copolymère selon l'invention et d'un agent émulsifiant.

De préférence, l'agent émulsifiant est choisi parmi ceux nécessaires dans la formulation finale, par exemple à la formulation de type OD finale. De façon particulièrement préféré, l'agent émulsifiant est un polyéthylène glycol esters d'acides gras. Il peut notamment s'agir du produit Alkamuls VO/2003, disponible auprès de la Société Rhodia.

Selon un mode de réalisation, l'invention concerne une composition concentrée consistant en un mélange d'un copolymère selon l'invention et d'un agent émulsifiant, en un ratio massique copolymère selon l'invention/agent émulsifiant compris entre 10:90 et 40:60, de préférence compris entre 15:85 et 35:65, par exemple de l'ordre de 20:80.

Selon un autre mode de réalisation, cette composition concentrée peut également comprendre en outre des charges additionnelles, par exemple des particules de silice, en une teneur inférieure à 5% en poids, par exemple inférieure à 2% en poids, par exemple inférieure à 1% en poids, par exemple comprise entre 0,1 et 0,5% en poids, par rapport au poids total de la composition.

A titre de charges additionnelles particulièrement adaptées à ce mode de réalisation, on peut notamment citer la silice commercialisée par la société Evonik sous la référence Aerosil® 200.

Selon un autre mode de réalisation, l'invention concerne également un concentré liquide à base d'un copolymère selon l'invention et d'une huile végétale ou d'origine végétale, avec ledit copolymère selon l'invention et ladite huile végétale ou d'origine végétale étant présents dans un ratio massique copolymère selon l'invention/huile végétale ou d'origine végétale compris entre 3:97 et 40:60, notamment entre 5:95 et 20:80, par exemple de l'ordre de 10:90.

A titre d'huile végétale ou d'origine végétale convenant particulièrement à ce mode de réalisation, on peut notamment citer l'huile de colza.

La présente invention va maintenant être décrite à l'aide d'exemples non limitatifs.

La figure 1 représente l'évolution de la contrainte en fonction de la vitesse de cisaillement d'une huile de colza à 25 et 54 °C et d'une composition d'huile de colza et d'un copolymère selon l'invention à 25 et 54 °C.

<u>Exemple 1 :</u> Préparation d'un copolymère selon l'invention

Dans un ballon tricol de 100 ml équipé d'un réfrigérant, d'une agitation mécanique (ancre), d'un bullage d'azote et d'un bain d'huile chauffant, on introduit 20g d'acrylate d'alkyle $C_{18}$-$C_{22}$ (acrylate de béhényle à 70%) et 16,4g d'huile de ricin. L'ensemble est chauffé à 80 °C puis la température est maintenue pendant 4h. A 80 °C on ajoute 1,29g de peroxyde de lauryle. Après 2h à 80 °C on rajoute 0,65g de peroxyde de lauryle.

Les caractéristiques du corps gras copolymérisé obtenu sont les suivantes :

- taux de greffage de l'huile de ricin : 12,4% ;
- taux de conversion en polyacrylate de béhényle : 100% ;
- Mw (mesurée par GPC relative avec calibration polystyrène) : 66150g/mol.

<u>Exemple 2 :</u> Préparation d'un copolymère selon l'invention

Dans un ballon tricol de 100ml équipé d'un réfrigérant, d'une agitation mécanique (ancre), d'un bullage d'azote et d'un bain d'huile chauffant, on introduit 20g d'acrylate d'alkyle $C_{18}$-$C_{22}$ (acrylate de béhényle à 70%), 21,4g d'huile de colza et 1,29g de peroxyde de lauryle. L'ensemble est chauffé à 90 °C pendant 4h.

Les caractéristiques du corps gras copolymérisé obtenu sont les suivantes :

- taux de greffage de l'huile de colza : 42,3% ;
- taux de conversion en polyacrylate de béhényle : 100% ;
- Mw (mesurée par GPC relative avec calibration polystyrène) :16230g/mol.

Exemple 3 : Préparation d'un copolymère selon l'invention

Dans un ballon tricol de 100ml équipé d'un réfrigérant, d'une agitation mécanique (ancre), d'un bullage d'azote et d'un bain d'huile chauffant, on introduit 20g d'acrylate d'alkyle $C_{18}$-$C_{22}$ (acrylate de béhényle à 70%) et 6,88g d'acide oléique. L'ensemble est chauffé à 80 °C puis la température est maintenue pendant 4h. A 80 °C on ajoute 1,29g de peroxyde de lauryle. Après 2h à 80 °C on rajoute 0.65g de peroxyde de lauryle.

Les caractéristiques du corps gras copolymérisé obtenu sont les suivantes :

- taux de greffage de l'acide oléique : 50,5% ;
- taux de conversion en polyacrylate de béhényle : 98,5%
- Mw (mesurée par GPC relative avec calibration polystyrène) : 517000 g/mol.

Exemple 4 : Préparation d'un copolymère non conforme à l'invention (contre-exemple)

Dans un réacteur verré de 1l équipé d'un réfrigérant, d'une agitation mécanique (ancre), d'un bullage d'azote et d'une double enveloppe où circule un fluide caloporteur, on introduit 154,41g d'acrylate de dodécyle et 254,93g d'huile de ricin. L'ensemble est chauffé à 60 °C et maintenu pendant 1h, A 60 °C on ajoute 7,24g de peroxyde de lauryle. Le milieu est ensuite chauffé à 70 °C et maintenu pendant 2h, puis à 75 °C pendant 2h, puis à 80 °C pendant 4h. Après 1h à 80 °C on ajoute 3,62g de peroxyde de lauryle.

Les caractéristiques du corps gras copolymérisé obtenu sont les suivantes :

- taux de greffage de l'huile de ricin : 6,4% ;
- taux de conversion en polyacrylate de lauryle : 98%
- Mw (mesurée par GPC relative avec calibration polystyrène) : 282000 g/mol.

Exemple 5 : Evaluation de la rhéologie du copolymère de l'exemple 1

Préparation des échantillons :

Dans un flacon de 50 mL muni d'un barreau aimanté de 25mm*8mm, 1,1g du copolymère de l'exemple 1 (soit 5,5% en masse) sont pesés, puis 18,9g d'huile de colza ajoutés (soit 94,5% en masse). Le mélange précédent est chauffé et est maintenu à une agitation de 300 tr/min. La solubilisation du copolymère de l'exemple 1 s'effectue à une température de 60 ± 2 °C. La solution obtenue est alors limpide et jaunâtre. Celle-ci est alors refroidie jusqu'à température ambiante sous agitation à 300 tr/min. La solution précédemment limpide s'épaissit. L'échantillon est alors stocké à température ambiante avant la réalisation de mesures rhéologiques.

Mesure du seuil par essai de fluage

La détermination des seuils à 25 et 54°C du mélange est réalisée à partir de tests de fluage sur un rhéomètre AR2000ex (TA INSTRUMENTS). Une géométrie cône-plan est utilisée avec un cône en aluminium d'angle 1deg 59min 2sec, de diamètre 60mm et de troncature 57$\mu$m.

Pour chacune des deux températures, un pré-cisaillement de 10$s^{-1}$ pendant 10s est effectué et l'échantillon est alors laissé au repos pendant 2 minutes. Des contraintes successives de 1,4 ; 1,6 ; 1,8 ; 2 ; 2,3 ; 2,5 ; 2,8 et 3 Pa à 25°C et de 0,1 ; 0,2 ; 0,3 ; 0,4 ; 0,5 ; 0,6 ; 0,7 ; 0,8 ; 1 ; 1,2 et 1,5 Pa à 54°C sont chacune appliquée pendant 2min. Pendant ce temps, la déformation du fluide qui en résulte est mesurée. Des tests de recouvrances de 6min sont réalisés à l'issue de chaque contrainte appliquée. Le taux de cisaillement pour chaque contrainte appliquée est alors obtenu en prenant la pente de la droite entre 80 et 120s.

La courbe donnant la contrainte versus le taux de cisaillement est alors tracée (Figure 1). La contrainte seuil, lorsqu'il y en a une, correspond à l'intersection des deux droites caractéristiques de la rupture de pente et est reportée dans le tableau 1 aux températures de 25°C et 54°C.

En complément, des mesures de viscosité à des vitesses de cisaillement de 1, 10 et 100 $s^{-1}$ ont également été réalisées avec le même cône que précédemment, ce qui permet d'évaluer l'augmentation de la viscosité du système à différents cisaillement et sa coulabilité à différentes températures.
Tous les résultats sont conciliés dans le tableau 1 suivant.

Tableau 1

| | Température 25°C | | | | Température 54°C | | | |
|---|---|---|---|---|---|---|---|---|
| Valeur de cisaillement | $1s^{-1}$ | $10s^{-1}$ | $100s^{-1}$ | Seuil (Pa) | $1s^{-1}$ | $10s^{-1}$ | $100s^{-1}$ | Seuil (Pa) |
| Système | Viscosité (Pa.s) | | | | Viscosité (Pa.s) | | | |
| Huile de colza | 0,06 | 0,06 | 0,06 | 0 | 0,02 | 0,02 | 0,02 | 0 |
| Huile de colza additivée de 5,5% du copolymère de l'exemple 1 | 5,70 | 0,97 | 0,36 | 2,5 | 0,71 | 0,15 | 0,08 | 0,8 |

L'huile de colza seule ne présente aucun seuil, aussi bien à 25 qu'à 54°C, et présente une viscosité constante à 1, 10 et $100s^{-1}$ (comportement Newtonien). L'ajout du copolymère de l'exemple 1 à l'huile de colza à hauteur de 5,5% en masse permet l'obtention d'un seuil important de l'ordre de 2,5 Pa à 25°C et surtout fait état de la présence d'un seuil important de 0,8Pa à la température de 54°C. La présence de tels seuils sur cette large gamme de températures permet avantageusement, dans des dispersions de composés solides dans l'huile le maintient en suspension d'actifs solides ainsi que et le maintien de la stabilité de la composition. De plus, l'addition du copolymère de l'exemple 1 fait état d'une augmentation significative de la viscosité du système à différents gradients de cisaillement, sans pour autant empêcher la « coulabilité » du système confirmant ainsi le comportement pseudo plastique du copolymère selon l'invention ; ce qui présente un avantage pour la préparation de diverses compositions, notamment des compositions cosmétiques ou encore pour ce qui est des revêtements.

Exemple 6: Evaluation de la stabilité d'une formulation complète réalisée avec un copolymère conforme à l'invention

Dans un bécher Pyrex de 250 ml, sont ajoutés à la spatule 6,6 g du copolymère de l'exemple 1 et à la pipette 24 g d'agent émulsifiant (polyéthylène glycol esters d'acides gras commercialisés par la Société Rhodia sous le nom Alkamuls VO/2003) et 84,36 g d'huile de colza. Ce mélange est alors chauffé sous une agitation de 300 tour/min jusqu'à solubilisation complète du copolymère de l'exemple 1, puis refroidi sous la même agitation jusqu'à température ambiante.
5,04 g de Nicosulfuron (actif solide) sont ensuite ajoutés puis s'en suit une homogénéisation à la spatule. Le mélange est ensuite passé 3 minutes à l'Ultra Turax T50 avec une pale double effet de D = 45 mm, à 6000 rpm avant d'être soumis à un broyage humide dans un MiniMotor Mill de marque Eiger Torrance LTD de 50 ml de capacité.
38 ml de billes SEPR ER 120 A, $ZrO_2/SiO_2$ de 0,8 à 1,25 mm de diamètre sont utilisées pour le broyage qui dure 10 mn à 3300 t/mn (graduation 7).
Il est contrôlé au microscope optique que toutes les particules sont de taille inférieure à 10 μm.

Tableau 2

| Formulation | Teneur en % massique |
|---|---|
| Nicosulfuron | 4,20 |
| Copolymère de l'exemple 1 | 5,50 |
| Alkamuls VO2003 | 20,00 |
| Huile de colza | 70,30 |

Différents tests métiers « types » de formulations agrochimiques sont réalisés sur cette formulation selon le tableau 2, pour s'assurer de la qualité et de la stabilité de cette dernière. On s'intéresse particulièrement à la stabilité de la dispersion en huile (séparation de phase : synérèse et/ou sédimentation) lors de tests de vieillissement (température ambiante ; -10°C pendant 7 jours, 54°C pendant 15 jours) ainsi qu'à sa viscosité. On s'intéresse également au comportement de la dispersion en huile lors de son basculement dans l'eau (pH, résidu tamis humide, mousse et stabilité de l'émulsion générée à différents temps (0 ; 0,5h ; 24h et redispersion à 24h puis attente 0,5h).
Les résultats sont présentés dans le tableau 3 :

| | | | Composition du tableau 2 | | |
|---|---|---|---|---|---|
| Stockage | | | 20 jours à température ambiante | 7 jours à -10°C | 15 jours à 54° C |
| Synérèse | | | 0% | 0% | 5,0% |
| Coulabilité | | | Coulable | Difficilement coulable à -10°C | Difficilement coulable à 54° C Légèrement jaune |
| Viscosité (mPas-1) | Viscosité Brookfield (20rpm) | | 1540(25° C) | 1860(24° C) | 2500(24° C) |
| pH (à 5 %) | pH | | 6,9 | 6,9 | 6,9 |
| Résidu tamis humide (inspiré de MT 185) | Rétention sur tamis 80 µm pour 5 % de composition diluée dans de l'eau de ville après agitation magnétique | | 0 % | 0 % | 0 % |
| Mousse (inspiré de MT 47.2) | Utilisation d'une éprouvette de 100 ml, ajout de 2 ml de composition à l'eau de ville, 30 inversions et observation de la quantité de mousse | | 0 immédiat | 0 immédiat | 0 immédiat |
| Stabilité de la dispersion (inspiré de MT 180) Formulation diluée à 2 %, à 30+/- 2°C, dans de l'eau de ville. Eprouvette de 100 ml utilisée, inversion 10 fois puis observation après : | Temps d'attente avant caractérisation | | | | |
| | 0 h | Aspect de l'émulsion | Emulsion blanche. Un peu de gras collé en surface | Emulsion blanche. Un peu de gras collé en surface | Emulsion très légèrement jaune. Quelques objets gras collés en surface |
| | 0,5 h | Crème (mm)<br>Huile (mm)<br>Sédiment (mm) | traces<br>0<br>0 | 0,5<br>0<br>0 | 0,5<br>0<br>0 |
| | 24 h | Crème (mm)<br>Huile (mm)<br>Sédiment (mm) | traces<br>0<br>0 | 0,5<br>0<br>0 | 0,5<br>0<br>0 |
| | | Re-dispersion | Un peu de gras collé en surface. | Un peu de gras collé en surface. | Quelques objets gras collés en surface. |
| | 24,5 h | Crème (mm)<br>Huile (mm)<br>Sédiment (mm) | 0,5<br>0<br>0 | 0,5<br>0<br>0 | 0,5<br>0<br>0 |

Tableau 3

Les résultats font état d'une formulation parfaitement stable à température ambiante après 20 jours et à -10°C après 7 jours puisqu'aucune séparation de phase n'apparait (ni synérèse, ni sédimentation). Le test de vieillissement accéléré (15 jours à 54°C) fait état d'une faible synérèse (5%) et de légères traces d'huile au fond sont observées. La formulation est coulable à température ambiante et difficilement coulable à -10 et 54°C même après retour à température ambiante.
Un pH de 6,9 est mesuré après dilution de 1% en masse de la composition dans de l'eau de ville. Le test de résidu tamis humide est bon, puisque aucun résidu n'est retenu sur le tamis. Le test de mousse est excellent, puisqu'aucune mousse résiduelle n'est présente après 30 inversions de l'éprouvette.
Les tests de stabilité de la dispersion est bon, avec obtention d'une émulsion stable, puisqu'aucune trace d'huile en surface, ni de sédiment n'est présent aux différents temps d'observation indiqués et un léger crémage de 0,5 ml maximum.
Cette crème est partiellement redispersable.
Ces résultats montrent que l'utilisation d'un copolymère selon l'invention permet de préparer des compositions comprenant une dispersion d'actifs phytosanitaires stables dans le temps et à température.
Bien évidemment ces résultats ne sauraient limiter l'invention à ce type d'actifs.
Ces résultats démontrent en effet, de manière plus générale, que les copolymères de l'invention permettent de préparer des compositions de tous types (agrochimiques, cosmétiques, pharmaceutiques, ...) comprenant au moins un actif, qui soient stables dans le temps et sur une large gamme de températures.

Exemple 7 : Exemple comparatif démontrant l'impact de la longueur de chaîne alkyle sur les performances

Mesures de seuil et de viscosité en utilisant un copolymère non conforme à l'invention (contre-exemple 4)

Préparation des échantillons :

Dans un flacon de 50 mL muni d'un barreau aimanté de 25mm*8mm, 1,1g du copolymère du contre exemple 4 (soit 5,5% en masse) sont pesés, puis 18,9g d'huile de colza ajoutés (soit 94,5% en masse). Le mélange précédent est chauffé et est maintenu à une agitation de 300 tr/min. La solubilisation du copolymère du contre exemple 4 s'effectue à une température de 60 ± 2°C. La solution obtenue est alors limpide et jaunâtre. Celle-ci est alors refroidie jusqu'à température ambiante sous agitation à 300 tr/min. L'échantillon est alors stocké à température ambiante avant la réalisation de mesures rhéologiques.

Mesure du seuil par essai de fluage

La détermination des seuils à 25 et 54°C du mélange est réalisée à partir de tests de fluage sur un rhéomètre AR2000ex (TA INSTRUMENTS). Une géométrie cône-plan est utilisée avec un cône en aluminium d'angle 1deg 59min 2sec, de diamètre 60mm et de troncature 57$\mu$m.
Pour chacune des deux températures, un pré-cisaillement de $10s^{-1}$ pendant 10s est effectué et l'échantillon est alors laissé au repos pendant 2 minutes. Des contraintes successives de 1,4 ; 1,6 ; 1,8 ; 2 ; 2,3 ; 2,5 ; 2,8 et 3 Pa à 25°C et de 0,1 ; 0,2 ; 0,3 ; 0,4 ; 0,5 ; 0,6 ; 0,7 ; 0,8 ; 1 ; 1,2 et 1,5 Pa à 54°C sont chacune appliquée pendant 2min. Pendant ce temps, la déformation du fluide qui en résulte est mesurée. Des tests de recouvrances de 6 min sont réalisés à l'issue de chaque contrainte appliquée. Le taux de cisaillement pour chaque contrainte appliquée est alors obtenu en prenant la pente de la droite entre 80 et 120s.
La courbe donnant la contrainte versus le taux de cisaillement est alors tracée. La contrainte seuil, lorsqu'il y en a une, correspond à l'intersection des deux droites caractéristiques de la rupture de pente, elle est reportée dans le tableau 4 aux températures de 25°C et 54°C.
En complément, des mesures de viscosité à des vitesses de cisaillement de 1, 10 et 100 $s^{-1}$ ont également été réalisées avec le même cône que précédemment, ce qui permet d'évaluer l'augmentation de la viscosité du système à différents cisaillement et sa coulabilité à différentes températures.
Tous les résultats sont conciliés dans le tableau 4 suivant.

Tableau 4

| | Température 25°C | | | | Température 54°C | | | |
|---|---|---|---|---|---|---|---|---|
| Valeur de cisaillement | $1s^{-1}$ | $10s^{-1}$ | $100s^{-1}$ | Seuil (Pa) | $1s^{-1}$ | $10s^{-1}$ | $100s^{-1}$ | Seuil (Pa) |
| Système | Viscosité (Pa.s) | | | | Viscosité (Pa.s) | | | |
| Huile de colza | 0,06 | 0,06 | 0,06 | 0 | 0,02 | 0,02 | 0,02 | 0 |

(suite)

| | Température 25°C | | | | Température 54°C | | | |
|---|---|---|---|---|---|---|---|---|
| Huile de colza additivée de 5,5% du copolymère du contre exemple 4 | 0,075 | 0,075 | 0,075 | 0 | 0,028 | 0,028 | 0,028 | 0 |

L'huile de colza seule ne présente aucun seuil, aussi bien à 25 qu'à 54°C, et présente une viscosité constante à 1, 10 et 100s$^{-1}$ (comportement Newtonien). L'ajout du copolymère du contre exemple 4 à l'huile de colza à hauteur de 5,5% en masse ne permet pas de changer les caractéristiques de l'huile de façon significative puisque la viscosité reste similaire et le comportement toujours Newtonien, alors que le seuil est nul à 25°C et à 54°C. L'absence de seuils sur cette large gamme de températures ne permet pas dans des dispersions de composés solides dans l'huile le maintient en suspension d'actifs solides comme illustré plus bas.

Evaluation de la stabilité d'une formulation complète réalisée avec un copolymère non conforme à l'invention (contre-exemple 4)

Dans un bécher Pyrex de 250 ml, sont ajoutés à la spatule 6,6 g d'un copolymère non conforme à l'invention (contre exemple 4) et à la pipette 24 g d'agent émulsifiant (polyéthylène glycol esters d'acides gras commercialisés par la Société Rhodia sous le nom d'Alkamuls VO2003) et 84,36 g d'huile de colza. Ce mélange est alors chauffé sous une agitation de 300 tour/min jusqu'à solubilisation complète du copolymère, puis refroidi sous la même agitation jusqu'à température ambiante. 5,04 g de Nicosulfuron (actif solide) sont ensuite ajoutés puis s'en suit une homogénéisation à la spatule. Le mélange est ensuite passé 3 minutes à l'Ultra Turax T50 avec une pale double effet de D = 45 mm, à 6000 rpm avant d'être soumis à un broyage humide dans un MiniMotor Mill de marque Eiger Torrance LTD de 50 ml de capacité.

38 ml de billes SEPR ER 120 A, $ZrO_2/SiO_2$ de 0,8 à 1,25 mm de diamètre sont utilisées pour le broyage qui dure 10 mn à 3300 t/mn (graduation 7).

Il est contrôlé au microscope optique que toutes les particules sont de taille inférieure à 10 $\mu$m.

Tableau 5

| Formulation | Teneur en % massique |
|---|---|
| Nicosulfuron | 4,20 |
| Copolymère du contre exemple 4 | 5,50 |
| Alkamuls VO2003 | 20,00 |
| Huile de colza | 70,30 |

Différents tests métiers « types » de formulations agrochimiques sont réalisés sur cette formulation selon le tableau 6, pour mesurer la stabilité de cette dernière.

On s'intéresse particulièrement à la stabilité de la dispersion en huile (séparation de phase : synérèse et/ou sédimentation) lors de tests de vieillissement (température ambiante ; 0°C pendant 7 jours, 54°C pendant 15 jours) ainsi qu'à sa viscosité. Les résultats sont présentés dans le tableau 6 :

Tableau 6

| Stockage | 14 jours à température ambiante | 7 jours à 0°C | 15 jours à 54° C |
|---|---|---|---|
| Synérèse (séparation de phase) | 68% | 56% | 68% |

Les résultats font état d'une formulation totalement instable à toutes les températures puisque l'actif (le Nicosulfuron) sédimente laissant place à une séparation de phase de plus de 50%. Aucun test de caractérisation supplémentaire n'a été réalisé car cette formulation n'est pas viable.

Exemple 8: Evaluation de la stabilité d'une formulation complète réalisée avec un copolymère conforme à l'invention

Dans un bécher Pyrex de 250 ml, sont ajoutés à la spatule 3,908 g du copolymère de l'exemple 1 et à la pipette 26,022g d'agent émulsifiant (polyéthylène glycol esters d'acides gras commercialisés par la Société Rhodia sous le nom

d'Alkamuls VO2003) et 91,926g d'huile de colza. 0,061g de silice Aerosil 200 (EVONIK) ainsi que 0,121g de carbonate de sodium sont ajoutés à la spatule. Ce mélange est alors chauffé sous une agitation de 300 tour/min jusqu'à solubilisation complète du copolymère selon l'invention, puis refroidi sous la même agitation jusqu'à température ambiante. 8,06 g de Tébuconazole (actif solide) sont ensuite ajoutés puis s'en suit une homogénéisation à la spatule. Le mélange est ensuite passé 3 minutes à l'Ultra Turax T50 avec une pale double effet de D = 45 mm, à 6000 rpm avant d'être soumis à un broyage humide dans un MiniMotor Mill de marque Eiger Torrance LTD de 50 ml de capacité.

38 ml de billes SEPR ER 120 A, $ZrO_2/SiO_2$ de 0,8 à 1,25 mm de diamètre sont utilisées pour le broyage qui dure 10 mn à 3300 t/mn (graduation 7).

Il est contrôlé au microscope optique que toutes les particules sont de taille inférieure à 10 μm.

Tableau 7

| Formulation | Teneur en % massique |
|---|---|
| Tébuconazole (98%) | 6,2 |
| Copolymère de l'exemple 1 | 3 |
| Alkamuls VO2003 | 20,00 |
| Huile de colza | 70,704 |
| Aerosil 200 | 0,046 |
| Carbonate de sodium | 0,09 |

Différents tests métiers « types » de formulations agrochimiques sont réalisés sur cette formulation selon le tableau 8, pour s'assurer de la qualité et de la stabilité de cette dernière. On s'intéresse particulièrement à la stabilité de la dispersion en huile (séparation de phase : synérèse et/ou sédimentation) lors de tests de vieillissement (température ambiante ; 0°C pendant 7 jours, 54°C pendant 15 jours) ainsi qu'à sa viscosité. On s'intéresse également au comportement de la dispersion en huile lors de son basculement dans l'eau (pH, résidu tamis humide, mousse et stabilité de l'émulsion générée à différents temps (0 ; 0,5h ; 24h et redispersion à 24h puis attente 0,5h).

Les résultats sont présentés dans le tableau 8 :

| | | | Composition du tableau 7 | | |
|---|---|---|---|---|---|
| Stockage | | | 20 jours à température ambiante | 7 jours à 0°C | 15 jours à 54° C |
| Synérèse | | | traces | 0% | 0% (changement de couleur et gradient de couleur sur 5%) |
| Coulabilité | | | Coulable | Coulable | Coulable |
| Viscosité (mPas-1) | Viscosité Brookfield (20rpm) | | 800 (25° C) | 600 (24° C) | 1300 (24° C) |
| pH (à 5 %) | pH | | 8,4 | 8,4 | 8,4 |
| Résidu tamis humide (inspiré de MT 185) | Rétention sur tamis 80 µm pour 5 % de composition diluée dans de l'eau de ville après agitation magnétique | | 0 % | 0 % | 0 % |
| Mousse (inspiré de MT 47.2) | Utilisation d'une éprouvette de 100 ml, ajout de 1 ml de composition à l'eau de ville, 30 inversions et observation de la quantité de mousse | | 0 immédiat | 0 immédiat | 0 immédiat |
| Stabilité de la dispersion (inspiré de MT 180) Formulation diluée à 1 %, à 30+/- 2°C, dans de l'eau CIPAC D. Eprouvette de 100 ml utilisée, inversion 10 fois puis observation après : | Temps d'attente avant caractérisation | | | | |
| | 0 h | Aspect de l'émulsion | Emulsion blanche. | Emulsion blanche. | Emulsion blanche légèrement plus claire |
| | 0,5 h | Crème (mm)<br>Huile (mm)<br>Sédiment (mm) | 0<br>0<br>0 | 0<br>0<br>0 | 0<br>0<br>0 |
| | 24 h | Crème (mm)<br>Huile (mm)<br>Sédiment (mm) | traces<br>0<br>0,1 | traces<br>0<br>0,1 | 1,5<br>0<br>0 |
| | | Re-dispersion | OK | OK | OK |
| | 24,5 h | Crème (mm)<br>Huile (mm)<br>Sédiment (mm) | 0<br>0<br>0 | 0<br>0<br>0 | 0<br>0<br>0 |

Tableau 8

Les résultats font état d'une formulation stable à température ambiante après 20 jours et à O°C après 7 jours puisqu'aucune séparation de phase n'apparait (ni synérèse, ni sédimentation). Le test de vieillissement accéléré (15 jours à 54°C) fait état d'un changement de couleur et d'un gradient de couleur sur les 5% du haut de l'échantillon. La formulation est coulable.

Un pH de 8,4 est mesuré après dilution de 5% en masse de la composition dans de l'eau de ville. Le test de résidu tamis humide est bon, puisque aucun résidu n'est retenu sur le tamis. Le test de mousse est excellent, puisqu'aucune mousse

résiduelle n'est présente après 30 inversions de l'éprouvette.

Les tests de stabilité de la dispersion est bon, avec obtention d'une émulsion stable, présentant après stockage à 54°C un léger crémage et quelques traces de dépôt à 0°C et à l'ambiante. Cette crème est redispersable.

Ces résultats montrent que l'utilisation du copolymère selon l'invention permet de préparer des compositions comprenant une dispersion d'actifs phytosanitaires stables dans le temps et à température.

Bien évidemment ces résultats ne sauraient limiter l'invention à ce type d'actifs.

Ces résultats démontrent en effet, de manière plus générale, que les copolymères de l'invention permettent de préparer des compositions de tous types (agrochimiques, cosmétiques, pharmaceutiques, ...) comprenant au moins un actif, qui soient stables dans le temps et sur une large gamme de températures

Exemple 9: Evaluation de la stabilité d'une formulation complète réalisée avec un copolymère conforme à l'invention

Dans un bécher Pyrex de 1000 ml, sont ajoutés à la spatule 45g du copolymère de l'exemple 1 et à la pipette 200g d'agent émulsifiant (polyéthylène glycol esters d'acides gras commercialisés par la Société Rhodia sous le nom d'Alkamuls VO2003) et 710,15g d'huile de colza. 0,85g de silice Aerosil 200 (EVONIK) ainsi que 1g de carbonate de sodium sont ajoutés à la spatule. Ce mélange est alors chauffé sous une agitation de 300 tour/min jusqu'à solubilisation complète du copolymère selon l'invention, puis refroidi sous la même agitation jusqu'à température ambiante. 1g d'antimousse Silcolapse RG22 (RHODIA) est alors ajouté sous agitation. 43 g de Nicosulfuron (actif solide, pureté 98%) sont ensuite ajoutés puis s'en suit une homogénéisation à la spatule. Le mélange est ensuite passé 3 minutes à l'Ultra Turax T50 avec une pale double effet de D = 45 mm, à 6000 rpm avant d'être soumis à un broyage humide dans un Vibro Mac LAB 2T de 1000 ml de capacité.

800 ml de billes SEPR ER 120 A, $ZrO_2/SiO_2$ de 0,8 à 1,25 mm de diamètre sont utilisées pour le broyage qui dure 20 mn.

Il est contrôlé au microscope optique que toutes les particules sont de taille inférieure à 10 μm.

Tableau 9

| Formulation | Teneur en % massique |
|---|---|
| Nicosulfuron (98%) | 4,3 |
| Copolymère de l'exemple 1 | 4,5 |
| Alkamuls VO2003 | 20,00 |
| Huile de colza | 71,015 |
| Aerosil 200 | 0,085 |
| Carbonate de sodium | 0,1 |
| Silcolapse RG22 | 0,1 |

Différents tests métiers « types » de formulations agrochimiques sont réalisés sur cette formulation selon le tableau 10, pour s'assurer de la qualité et de la stabilité de cette dernière.

On s'intéresse particulièrement à la stabilité de la dispersion en huile (séparation de phase : synérèse et/ou sédimentation) lors de tests de vieillissement (température ambiante ; 0°C pendant 7 jours, 54°C pendant 15 jours) ainsi qu'à sa viscosité. On s'intéresse également au comportement de la dispersion en huile lors de son basculement dans l'eau (pH, résidu et stabilité de l'émulsion générée à différents temps (0 ; 1h).

Les résultats sont présentés dans le tableau 10 :

<table>
<tr><td colspan="3">Stockage</td><td>30 jours à température ambiante</td><td>7 jours à 0°C</td><td>15 jours à 54°C</td></tr>
<tr><td colspan="3">Synérèse</td><td>0%</td><td>0%</td><td>12%</td></tr>
<tr><td colspan="3">Coulabilité</td><td>Coulable</td><td>Coulable</td><td>Coulable</td></tr>
<tr><td>Viscosité (mPas-1)</td><td colspan="2">Viscosité Brookfield (20rpm)</td><td>960 (20°C)</td><td>980 (20°C)</td><td>1140 (20°C)</td></tr>
<tr><td>pH (à 5 %)</td><td colspan="2">pH</td><td>5,2</td><td>5,2</td><td>5,1</td></tr>
<tr><td rowspan="3">Stabilité de la dispersion (inspiré de MT 180) Formulation diluée à 1 %, à 30+/- 2°C, dans eau CIPAC D. Eprouvette de 100 ml utilisée, inversion 10 fois puis observation après :</td><td colspan="2">Temps d'attente avant caractérisation</td><td colspan="3"></td></tr>
<tr><td>0 h</td><td>Aspect de l'émulsion</td><td>Emulsion blanche.</td><td>Emulsion blanche.</td><td>Emulsion blanche</td></tr>
<tr><td>1 h</td><td>Crème (mm)<br>Huile (mm)<br>Sédiment (mm)</td><td>1<br>0<br>0</td><td>1<br>0<br>0</td><td>2<br>0<br>0</td></tr>
</table>

Tableau 10

Les résultats font état d'une formulation stable à température ambiante après 30 jours et à 0°C après 7 jours puisqu'aucune séparation de phase n'apparait (ni synérèse, ni sédimentation). Le test de vieillissement accéléré (15 jours à 54°C) fait état d'une séparation de phase limitée sur les 12% du haut de l'échantillon, que nous pouvons re-homogénéiser après mélange. La formulation est coulable.
Un pH de 5,2 est mesuré après dilution de 5% en masse de la composition dans de l'eau CIPAC D.
Les tests de stabilité de la dispersion est bon, avec obtention d'une émulsion stable, présentant un léger crémage et quelques traces de dépôt. Cette crème est redispersable.

Ces résultats montrent que l'utilisation du copolymère selon l'invention permet de préparer des compositions comprenant une dispersion d'actifs phytosanitaires stables dans le temps et à température.

Exemple 10 : Evaluation de la stabilité d'une formulation complète réalisée avec un copolymère conforme à l'invention

Dans un bécher Pyrex de 1000 ml, sont ajoutés à la spatule 45g du copolymère de l'exemple 1 et à la pipette 200g d'agent émulsifiant (polyéthylène glycol esters d'acides gras commercialisés par la Société Rhodia sous le nom d'Alkamuls VO2003) et 699,15g d'huile de colza. 0,85g de silice Aerosil 200 (EVONIK) ainsi que 1g de carbonate de sodium sont ajoutés à la spatule. Ce mélange est alors chauffé sous une agitation de 300 tour/min jusqu'à solubilisation complète du copolymère selon l'invention, puis refroidi sous la même agitation jusqu'à température ambiante. 1g d'anti-mousse Silcolapse RG22 (RHODIA) est alors ajouté sous agitation. 53 g de Flufenoxuron (actif solide, pureté 98%) sont ensuite ajoutés puis s'en suit une homogénéisation à la spatule. Le mélange est ensuite passé 3 minutes à l'Ultra Turax T50 avec une pale double effet de D = 45 mm, à 6000 rpm avant d'être soumis à un broyage humide dans un Vibro Mac LAB 2T de 1000 ml de capacité.
800 ml de billes SEPR ER 120 A, $ZrO_2/SiO_2$ de 0,8 à 1,25 mm de diamètre sont utilisées pour le broyage qui dure 20 mn. Il est contrôlé au microscope optique que toutes les particules sont de taille inférieure à 10 μm.

Tableau 11

| Formulation | Teneur en % massique |
|---|---|
| Flufenoxuron (98%) | 5,3 |
| Copolymère de l'exemple 1 | 4,5 |
| Alkamuls VO2003 | 20,00 |
| Huile de colza | 69,915 |

(suite)

| Formulation | Teneur en % massique |
|---|---|
| Aerosil 200 | 0,085 |
| Carbonate de sodium | 0,1 |
| Silcolapse RG22 | 0,1 |

Différents tests métiers « types » de formulations agrochimiques sont réalisés sur cette formulation selon le tableau 12, pour s'assurer de la qualité et de la stabilité de cette dernière.
On s'intéresse particulièrement à la stabilité de la dispersion en huile (séparation de phase : synérèse et/ou sédimentation) lors de tests de vieillissement (température ambiante ; 0°C pendant 7 jours, 54°C pendant 15 jours) ainsi qu'à sa viscosité. On s'intéresse également au comportement de la dispersion en huile lors de son basculement dans l'eau (pH, et stabilité de l'émulsion générée à différents temps (0 ; 1h).
Les résultats sont présentés dans le tableau 12 :

| Stockage | | | 30 jours à température ambiante | 7 jours à 0°C | 15 jours à 54°C |
|---|---|---|---|---|---|
| Synérèse | | | 0% | 0% | 2% |
| Coulabilité | | | Coulable | Coulable | Gel |
| Viscosité (mPas-1) | Viscosité Brookfield (20rpm) | | 860 (20°C) | 900 (20°C) | 6000 (20°C) |
| pH (à 5 %) | pH | | 8,1 | 8,3 | 8,6 |
| Stabilité de la dispersion (inspiré de MT 180) Formulation diluée à 1 %, à 30+/- 2°C, dans eau CIPAC D. Eprouvette de 100 ml utilisée, inversion 10 fois puis observation après : | Temps d'attente avant caractérisation | | | | |
| | 0 h | Aspect de l'émulsion | Emulsion blanche. | Emulsion blanche. | Emulsion blanche |
| | 1 h | Crème (mm)<br>Huile (mm)<br>Sédiment (mm) | 0<br>0<br>0 | 0<br>0<br>0 | 0<br>0<br>0 |

Tableau 12

Les résultats font état d'une formulation stable à température ambiante après 30 jours et à 0°C après 7 jours puisqu'aucune séparation de phase n'apparait (ni synérèse, ni sédimentation). Le test de vieillissement accéléré (15 jours à 54°C) fait état d'une séparation de phase quasi nulle (2% sur le haut de l'échantillon), néanmoins la formulation apparaît gélifiée. Il est nécessaire de l'agiter pour qu'elle redevienne coulable et cette dernière reste très visqueuse ;
Un pH de 8,1 est mesuré après dilution de 5% en masse de la composition dans de l'eau CIPAC D.
Les tests de stabilité de la dispersion est bon, avec obtention d'une émulsion stable, présentant aucun crémage, ni traces de dépôt.
Ces résultats montrent que l'utilisation du copolymère selon l'invention permet de préparer des compositions comprenant une dispersion d'actifs phytosanitaires stables dans le temps et à température.

<u>Exemple 11 :</u> Réalisation d'une formulation concentrée liquide de mise en oeuvre aisée contenant un copolymère selon l'invention et un émulsifiant.

Ce concentré peut être notamment utilisable pour réaliser une formulation complète, de type OD par exemple. Dans un bécher Pyrex de 1000 ml, sont ajoutés à la spatule 215,3g du copolymère de l'exemple 1 et 781,3g d'agent émulsifiant (polyéthylène glycol esters d'acides gras commercialisés par la Société Rhodia sous le nom d'Alkamuls

VO2003) et 3,4g de silice Aérosil 200 (EVONIK). Ce mélange est alors chauffé sous une agitation de 300 tour/min jusqu'à solubilisation complète du copolymère selon l'invention, puis refroidi sous la même agitation jusqu'à température ambiante.

Tableau 13

| Formulation | Teneur en % massique |
|---|---|
| Copolymère de l'exemple 1 | 21,53 |
| Alkamuls VO2003 | 78,13 |
| Aerosil 200 | 0,34 |

Une formulation concentrée liquide stable et facilement manipulable est obtenue. Ce concentré liquide contenant les deux fonctionnalités (d'une part lagent rhéologique (copolymère selon l'invention) et d'autre part l'émulsifiant (Alkamuls VO2003)) peut être stabilisé si nécessaire par l'ajout d'un peu de silice. La viscosité mesurée de ce mélange est de l'ordre de 2500cP à 20rpm et 25°C et est stable à l'ambiante, 0°C et 54°C.

Une quantité adéquate de ce concentré peut-être utilisée pour réaliser dans un deuxième temps une formulation complète, par exemple de type OD, à l'image de celles décrites dans les exemples 8 à 10.

## Revendications

**1.** Composition émulsifiable par mélange avec de l'eau, comprenant :

- un milieu apolaire ;
- un composé, par exemple un actif phytosanitaire, dispersé au sein dudit milieu apolaire ;
- un copolymère dont le squelette est obtenu par polymérisation radicalaire :

  • d'un corps gras (A) comprenant des insaturations, choisi parmi les huiles végétales ou les huiles d'origine végétales choisies parmi les triglycérides d'acides gras saturés ou insaturés comprenant au moins 12 atomes de carbone et de préférence de 14 à 22 atomes de carbone ; les esters des triglycérides d'acides gras saturés ou insaturés comprenant au moins 12 atomes de carbone et de préférence de 14 à 22 atomes de carbone, et notamment leurs esters méthyliques et éthyliques ; ou les huiles animale ou d'origine animales, par exemple les huiles de poissons ; ou leurs mélanges ; et
  • d'au moins un monomère (B) comprenant au moins une fonction polymérisable par polymérisation radicalaire et comprenant au moins une chaîne alkyle, linéaire ou ramifiée comprenant de 16 à 44 atomes de carbone; et

- un agent émulsifiant.

**2.** Composition selon la revendication 1, avec le monomère (B) dudit copolymère comprenant une chaîne alkyle, linéaire ou ramifiée, comprenant au moins 18 atomes de carbone, par exemple au moins 20 atomes de carbone, notamment 22 atomes de carbone ou 44 atomes de carbone.

**3.** Composition selon la revendication 1 ou 2, avec le corps gras (A) dudit copolymère étant choisi parmi l'huile de colza, l'huile de soja, l'huile de maïs, l'huile de ricin, l'huile d'arachide, l'huile de beurre, l'huile de graine de coton, l'huile de lin, l'huile de noix de coco, l'huile d'olive, l'huile de palme, l'huile de pépin de raisin, l'huile de coprah et leurs mélanges.

**4.** Composition selon l'une des revendications 1 à 3, avec le monomère (B) dudit copolymère étant choisi parmi :

- les acrylates d'alkyle ;
- les méthacrylates d'alkyle ;
- les acrylamides d'alkyle ;
- les méthacrylamides d'alkyle ;
- les vinyles d'alkyle, notamment les allyles d'alkyles ou les vinyléthers d'alkyle ; et
- les styrènes d'alkyle ;

dans lesquels l'alkyle est une chaîne, linéaire ou ramifiée, comprenant de préférence au moins 18 atomes de carbones, par exemple au moins 20 atomes de carbone, notamment 22 atomes de carbone ou 44 atomes de carbone.

**5.** Composition selon l'une des revendications 1 à 4, avec le monomère (B) dudit copolymère étant choisi parmi un acrylate d'alkyle dans lequel la chaine alkyle comprend 22 atomes de carbone ou un acrylate d'alkyle dans lequel la chaine alkyle comprend 44 atomes de carbone.

**6.** Composition selon l'une des revendications 1 à 5, avec ledit copolymère ayant un taux de greffage du corps gras (A) compris entre 5 et 80%, selon l'une des formules suivantes (eq1) ou (eq3) selon que le corps gras (A) comprend respectivement uniquement des insaturations (eq1), ou à la fois des insaturations et des fonctions hydroxyles (eq3):

$$taux\ de\ greffage = \left(\frac{A2}{A1}\right) \quad \text{(eq1)}$$

$$taux\ de\ greffage = \left(\frac{A2}{A1}\right) + \left(\frac{B2}{B1}\right) \quad \text{(eq3)}$$

dans laquelle :

$$\left(\frac{A2}{A1}\right) = \left(\frac{nombre\ d'insaturations\ polymérisées}{nombre\ d'insaturations\ initiales}\right)$$

$$\left(\frac{B2}{B1}\right) = \left(\frac{nombre\ de\ fonctions\ hydroxyles\ substituées}{nombre\ de\ fonctions\ hydroxyles\ initiales}\right)$$

**7.** Composition selon l'une des revendications 1 à 6, avec ledit copolymère ayant une masse moléculaire comprise entre 10 000 et $1.10^6$ g/mol, de préférence comprise entre 15 000 et 500 000 g/mol, plus préférentiellement comprise entre 20 000 et 250 000 g/mol, par exemple entre 25 000 et 80 000 g/mol.

**8.** Composition selon l'une quelconque des revendications 1 à 7, dans laquelle le milieu apolaire est choisi parmi :

- les triglycérides d'acides gras saturés ou insaturés comprenant au moins 12 atomes de carbone et de préférence de 14 à 22 atomes de carbone ; en particulier des triglycérides de synthèse ou de préférence des triglycérides naturels, tels que les huiles végétales ou d'origine végétales, du type huile de colza, huile de soja, huile d'arachide, huile de beurre, huile de graine de coton, huile de lin, huile de noix de coco, huile d'olive, huile de palme, huile de pépin de raisin, huile de ricin, huile de coprah, ou les huiles animales ou d'origine animales, par exemple huiles de poisson, notamment les huiles de poisson comprenant des oméga 3 ;
- les esters des triglycérides d'acides gras saturés ou insaturés comprenant au moins 12 atomes de carbone et de préférence de 14 à 22 atomes de carbone, notamment leurs esters méthyliques et éthyliques ;
- les coupes pétrolières aromatiques ;
- les solvants aromatiques, par exemple anisole, toluène ;
- les composés terpéniques, par exemple D-limonène, L-limonène ;
- les mélanges de diesters succinate/adipate/glutarate de diméthyle ;
- les hydrocarbures aliphatiques comprenant au moins 6 atomes de carbone par exemple isooctane, kerosène, essence, essence diesel, huiles minérales, notamment huile de paraffine ; les huiles lubrifiantes ; ou
- leurs mélanges.

**9.** Composition selon les revendications 1 à 8, dans laquelle le milieu apolaire comprend, voire est, un mélange de triglycérides, par exemple une huile végétale ou d'origine végétale choisie parmi l'huile de colza, l'huile de soja, l'huile de maïs, l'huile de ricin, l'huile d'arachide, l'huile de beurre, l'huile de graine de coton, l'huile de lin, l'huile de noix de coco, l'huile d'olive, l'huile de palme, l'huile de pépin de raisin, l'huile de coprah et leurs mélanges.

**10.** Composition selon les revendications 1 à 9, comprenant entre 0,5 et 40% en poids, par exemple entre 1 et 20% en poids, de préférence entre 2 et 10 en poids, par exemple entre 3 et 8% en poids, de copolymère par rapport au poids total de la composition.

**11.** Composition selon les revendications 1 à 10, dans laquelle le milieu apolaire est une huile végétale ou un mélange d'huiles végétales et l'émulsifiant est un polyéthylène glycol esters d'acides gras.

**12.** Composition selon les revendications 1 à 11, comprenant entre 3 et 30% en poids d'agent émulsifiant par rapport au poids total de la composition.

**13.** Procédé de préparation d'une composition selon l'une des revendications 1 à 12, choisi parmi :

- un procédé (P1) comprenant les étapes suivantes :

(i) on mélange le milieu apolaire et l'agent émulsifiant ; puis
(ii) on ajoute le copolymère ; puis
(iii) on ajoute le composé à disperser ; ou

- un procédé (P2) comprenant les étapes suivantes :

(a) on mélange le milieu apolaire, l'agent émulsifiant et le composé à disperser ; puis
(b) on ajoute à chaud, le copolymère solubilisé dans du milieu apolaire ; ou

- un procédé (P3) comprenant les étapes suivantes :

(I) on mélange le milieu apolaire et le composé à disperser ; puis
(II) on ajoute à chaud le copolymère solubilisé dans du milieu apolaire et l'agent émulsifiant.

**14.** Utilisation d'une composition selon l'une des revendications 1 à 12 pour former une émulsion de type huile-dans-l'eau par mélange de la composition avec une phase aqueuse.

**15.** Composition concentrée comprenant un copolymère dont le squelette est obtenu par polymérisation radicalaire :

• d'un corps gras (A) comprenant des insaturations, choisi parmi les huiles végétales ou les huiles d'origine végétales choisies parmi les triglycérides d'acides gras saturés ou insaturés comprenant au moins 12 atomes de carbone et de préférence de 14 à 22 atomes de carbone ; les esters des triglycérides d'acides gras saturés ou insaturés comprenant au moins 12 atomes de carbone et de préférence de 14 à 22 atomes de carbone, et notamment leurs esters méthyliques et éthyliques ; ou les huiles animale ou d'origine animales, par exemple les huiles de poissons ; ou leurs mélanges ; et
• d'au moins un monomère (B) comprenant au moins une fonction polymérisable par polymérisation radicalaire et comprenant au moins une chaîne alkyle, linéaire ou ramifiée comprenant de 16 à 44 atomes de carbone

et un agent émulsifiant, de préférence choisi parmi les polyéthylène glycol esters d'acides gras.

**16.** Composition concentrée selon la revendication 15, avec ledit copolymère étant tel que défini en revendications 2 à 7.

## Patentansprüche

**1.** Zusammensetzung, die durch Mischen mit Wasser emulgierbar ist und Folgendes umfasst:

- ein apolares Medium,
- eine in dem apolaren Medium dispergierte Verbindung, zum Beispiel einen Pflanzenschutzwirkstoff,
- ein Copolymer, dessen Grundgerüst durch radikalische Polymerisation von Folgendem erhalten wird:

• einem Fettkörper (A), der Ungesättigtheiten umfasst, ausgewählt aus Pflanzenölen oder Ölen pflanzlichen Ursprungs, die aus Triglyceriden gesättigter oder ungesättigter Fettsäuren mit mindestens 12 Kohlenstoffatomen und vorzugsweise mit 14 bis 22 Kohlenstoffatomen; Estern von Triglyceriden gesättigter oder

ungesättigter Fettsäuren mit mindestens 12 Kohlenstoffatomen und vorzugsweise mit 14 bis 22 Kohlenstoffatomen und insbesondere deren Methyl- und Ethylestern ausgewählt sind, oder tierischen Ölen oder Ölen tierischen Ursprungs, zum Beispiel Fischölen, oder deren Gemischen, und

• mindestens einem Monomer (B), das mindestens eine durch radikalische Polymerisation polymerisierbare Funktion umfasst und mindestens eine gerade oder verzweigte Alkylkette mit 16 bis 44 Kohlenstoffatomen umfasst, und

• einen Emulgator.

**2.** Zusammensetzung nach Anspruch 1, wobei das Monomer (B) des Copolymers eine gerade oder verzweigte Alkylkette mit mindestens 18 Kohlenstoffatomen, zum Beispiel mit mindestens 20 Kohlenstoffatomen, insbesondere mit 22 Kohlenstoffatomen oder 44 Kohlenstoffatomen umfasst.

**3.** Zusammensetzung nach Anspruch 1 oder 2, wobei der Fettkörper (A) des Copolymers aus Rapsöl, Sojaöl, Maisöl, Ricinusöl, Erdnussöl, Butteröl, Baumwollsamenöl, Leinöl, Kokosnussöl, Olivenöl, Palmöl, Traubenkernöl, Kopraöl und deren Gemischen ausgewählt ist.

**4.** Zusammensetzung nach einem der Ansprüche 1 bis 3, wobei das Monomer (B) des Copolymers aus den Folgenden ausgewählt ist:

- Alkylacrylaten,
- Alkylmethacrylaten,
- Alkylacrylamiden,
- Alkylmethacrylamiden,
- Alkylvinylen, insbesondere Alkylallylen oder Alkylvinylethern, und
- Alkylstyrolen,

wobei das Alkyl jeweils eine gerade oder verzweigte Kette ist, die vorzugsweise mindestens 18 Kohlenstoffatome, zum Beispiel mindestens 20 Kohlenstoffatome, insbesondere 22 Kohlenstoffatome oder 44 Kohlenstoffatome umfasst.

**5.** Zusammensetzung nach einem der Ansprüche 1 bis 4, wobei das Monomer (B) des Copolymers aus einem Alkylacrylat, wobei die Alkylkette 22 Kohlenstoffatome umfasst, oder einem Alkylacrylat, wobei die Alkylkette 44 Kohlenstoffatome umfasst, ausgewählt ist.

**6.** Zusammensetzung nach einem der Ansprüche 1 bis 5, wobei das Copolymer einen Pfropfungsgrad des Fettkörpers (A) zwischen 5 und 80% gemäß einer der folgenden Formeln (G11) oder (G13) besitzt, je nachdem, ob der Fettkörper (A) nur Ungesättigtheiten (G11) oder gleichzeitig Ungesättigtheiten und Hydroxylfunktionen enthält (G13):

$$Pfropfungsgrad = \left(\frac{A2}{A1}\right)\ldots(Gl1)$$

$$Pfropfungsgrad = \left(\frac{A2}{A1}\right) + \left(\frac{B2}{B1}\right)\ldots(Gl3),$$

wobei

$$\left(\frac{A2}{A1}\right) = \left(\frac{Anzahl\ldots der\ldots polymerisierten\ldots Ungesättigtheiten}{Anzahl\ldots der\ldots anfänglichen\ldots Ungesättigheiten}\right)$$

$$\left(\frac{B2}{B1}\right) = \left(\frac{Anzahl\ldots der\ldots substituierten\ldots Hydroxylfunktionen}{Anzahl\ldots der\ldots anfänglichen\ldots Hydroxylfunktionen}\right)$$

7. Zusammensetzung nach einem der Ansprüche 1 bis 6, wobei das Copolymer eine Molekülmasse zwischen 10000 und $1.10^6$ g/mol, vorzugsweise zwischen 15000 und 500000 g/mol, stärker bevorzugt zwischen 20000 und 250000 g/mol, zum Beispiel zwischen 25000 und 80000 g/mol besitzt.

8. Zusammensetzung nach einem der Ansprüche 1 bis 7, wobei das apolare Medium aus den Folgenden ausgewählt ist:

- Triglyceriden gesättigter oder ungesättigter Fettsäuren mit mindestens 12 Kohlenstoffatomen und vorzugsweise mit 14 bis 22 Kohlenstoffatomen; insbesondere synthetischen Triglyceriden oder vorzugsweise natürlichen Triglyceriden, wie Pflanzenöle oder Öle pflanzlichen Ursprungs des Typs Rapsöl, Sojaöl, Erdnussöl, Butteröl, Baumwollsamenöl, Leinöl, Kokosnussöl, Olivenöl, Palmöl, Traubenkernöl, Ricinusöl, Kopraöl, oder tierische Öle oder Öle tierischen Ursprungs, zum Beispiel Fischöle, insbesondere Fischöle, die Omega-3 umfassen;
- Estern von Triglyceriden gesättigter oder ungesättigter Fettsäuren mit mindestens 12 Kohlenstoffatomen und vorzugsweise mit 14 bis 22 Kohlenstoffatomen, insbesondere deren Methyl- und Ethylester;
- aromatischen Erdölfraktionen;
- aromatischen Lösungsmitteln, zum Beispiel Anisol, Toluol;
- Terpenverbindungen, zum Beispiel D-Limonen, L-Limonen;
- Gemischen von Dimethylsuccinat-/-adipat-/-glutarat-Diestern;
- aliphatischen Kohlenwasserstoffen mit mindestens 6 Kohlenstoffatomen, zum Beispiel Isooctan, Kerosin, Benzin, Dieselkraftstoff, Mineralöle, insbesondere Paraffinöl; Schmieröle; oder
- deren Gemischen.

9. Zusammensetzung nach den Ansprüchen 1 bis 8, wobei das apolare Medium ein Gemisch von Triglyceriden, zum Beispiel ein Pflanzenöl oder ein Öl pflanzlichen Ursprungs, ausgewählt aus Rapsöl, Sojaöl, Maisöl, Ricinusöl, Erdnussöl, Butteröl, Baumwollsamenöl, Leinöl, Kokosnussöl, Olivenöl, Palmöl, Traubenkernöl, Kopraöl und deren Gemische, umfasst oder sogar ist.

10. Zusammensetzung nach den Ansprüchen 1 bis 9, die zwischen 0,5 und 40 Gew.-%, zum Beispiel zwischen 1 und 20 Gew.-%, vorzugsweise zwischen 2 und 10 Gew.-%, zum Beispiel zwischen 3 und 8 Gew.-% Copolymer, bezogen auf das Gesamtgewicht der Zusammensetzung, umfasst.

11. Zusammensetzung nach den Ansprüchen 1 bis 10, wobei das apolare Medium ein Pflanzenöl oder ein Gemisch von Pflanzenölen ist und der Emulgator ein Fettsäurepolyethylenglykolester ist.

12. Zusammensetzung nach den Ansprüchen 1 bis 11, die zwischen 3 und 30 Gew.-% Emulgator, bezogen auf das Gesamtgewicht der Zusammensetzung, umfasst.

13. Verfahren zur Herstellung einer Zusammensetzung nach einem der Ansprüche 1 bis 12, das aus Folgenden ausgewählt ist:

- einem Verfahren (P1), das die folgenden Schritte umfasst:

  (i) man mischt das apolare Medium und den Emulgator und
  (ii) gibt das Copolymer hinzu und
  (iii) gibt die zu dispergierende Verbindung hinzu, oder

- einem Verfahren (P2), das die folgenden Schritte umfasst:

  (a) man mischt das apolare Medium, den Emulgator und die zu dispergierende Verbindung und
  (b) gibt im Heißen das in dem apolaren Medium solubilisierte Copolymer hinzu, oder

- einem Verfahren (P3), das die folgenden Schritte umfasst:

  (i) man mischt das apolare Medium und die zu dispergierende Verbindung und
  (ii) gibt im Heißen das in dem apolaren Medium solubilisierte Copolymer und den Emulgator hinzu.

14. Verwendung einer Zusammensetzung nach einem der Ansprüche 1 bis 12 zur Herstellung einer Emulsion des Öl-in-Wasser-Typs durch Mischen der Zusammensetzung mit einer wässrigen Phase.

**15.** Konzentrierte Zusammensetzung, umfassend ein Copolymer, dessen Grundgerüst durch radikalische Polymerisation von Folgendem erhalten wird:

• einem Fettkörper (A), der Ungesättigtheiten umfasst, ausgewählt aus Pflanzenölen oder Ölen pflanzlichen Ursprungs, die aus Triglyceriden gesättigter oder ungesättigter Fettsäuren mit mindestens 12 Kohlenstoffatomen und vorzugsweise mit 14 bis 22 Kohlenstoffatomen; Estern von Triglyceriden gesättigter oder ungesättigter Fettsäuren mit mindestens 12 Kohlenstoffatomen und vorzugsweise mit 14 bis 22 Kohlenstoffatomen und insbesondere deren Methyl- und Ethylestern ausgewählt sind; oder tierischen Ölen oder Ölen tierischen Ursprungs, zum Beispiel Fischölen, oder deren Gemischen, und
• mindestens einem Monomer (B), das mindestens eine durch radikalische Polymerisation polymerisierbare Funktion umfasst und mindestens eine gerade oder verzweigte Alkylkette mit 16 bis 44 Kohlenstoffatomen umfasst, und

einen Emulgator, der vorzugsweise aus Polyethylenglykolestern von Fettsäuren ausgewählt ist.

**16.** Konzentrierte Zusammensetzung nach Anspruch 15, wobei das Copolymer wie in einem der Ansprüche 2 bis 7 definiert ist.

## Claims

**1.** Composition that is emulsifiable by mixing with water, comprising:

- a nonpolar medium;
- a compound, for example a phytosanitary active agent, dispersed within said nonpolar medium;
- a copolymer of which the backbone is obtained by radical polymerization:

  • of a fatty substance (A) comprising unsaturations, selected from vegetable oils or oils of vegetable origin, selected from triglycerides of saturated or unsaturated fatty acids comprising at least 12 carbon atoms and preferably from 14 to 22 carbon atoms; esters of triglycerides of saturated or unsaturated fatty acids comprising at least 12 carbon atoms and preferably from 14 to 22 carbon atoms, and in particular the methyl and ethyl esters thereof; or animal oils or oils of animal origin, for example fish oils; or mixtures thereof; and

- of at least one monomer (B) comprising at least one function that can be polymerized by radical polymerization and that comprises at least one linear or branched alkyl chain comprising from 16 to 44 carbon atoms; and
- an emulsifier.

**2.** Composition according to Claim 1, with the monomer (B) of said copolymer comprising a linear or branched alkyl chain comprising at least 18 carbon atoms, for example at least 20 carbon atoms, in particular 22 carbon atoms or 44 carbon atoms.

**3.** Composition according to Claim 1 or 2, with the fatty substance (A) of said copolymer being selected from rapeseed oil, soybean oil, corn oil, castor oil, groundnut oil, butter oil, cottonseed oil, linseed oil, coconut oil, olive oil, palm oil, grapeseed oil, copra oil, and mixtures thereof.

**4.** Composition according to one of Claims 1 to 3, with the monomer (B) of said copolymer being selected from:

- alkyl acrylates;
- alkyl methacrylates;
- alkylacrylamides;
- alkylmethacrylamides;
- alkyl vinyls, in particular alkyl allyls or alkyl vinyl ethers; and
- alkylstyrenes;

in which the alkyl is a linear or branched chain preferably comprising at least 18 carbon atoms, for example at least 20 carbon atoms, in particular 22 carbon atoms or 44 carbon atoms.

**5.** Composition according to one of Claims 1 to 4, with the monomer (B) of said copolymer being selected from an

alkyl acrylate in which the alkyl chain comprises 22 carbon atoms or an alkyl acrylate in which the alkyl chain comprises 44 carbon atoms.

6. Composition according to one of Claims 1 to 5, with said copolymer having a degree of grafting of the fatty substance (A) of between 5% and 80%, according to one of the following formulae (eq1) or (eq3) depending on whether the fatty substance (A) comprises, respectively, only unsaturations (eq1), or both unsaturations and hydroxyl functions (eq3):

$$degree\ of\ grafting = \left(\frac{A2}{A1}\right) \quad \text{(eq1)}$$

$$degree\ of\ grafting = \left(\frac{A2}{A1}\right) + \left(\frac{B2}{B1}\right) \quad \text{(eq3)}$$

in which:

$$\left(\frac{A2}{A1}\right) = \left(\frac{number\ of\ unsaturations\ polymerized}{number\ of\ initial\ unsaturations}\right)$$

$$\left(\frac{B2}{B1}\right) = \left(\frac{number\ of\ hydroxyl\ functions\ substituted}{number\ of\ initial\ hydroxyl\ functions}\right)$$

7. Composition according to one of Claims 1 to 6, with said copolymer having a molecular weight of between 10 000 and $1 \times 10^6$ g/mol, preferably between 15 000 and 500 000 g/mol, more preferentially between 20 000 and 250 000 g/mol, for example between 25 000 and 80 000 g/mol.

8. Composition according to any one of Claims 1 to 7, wherein the nonpolar medium is selected from:

- triglycerides of saturated or unsaturated fatty acids comprising at least 12 carbon atoms and preferably from 14 to 22 carbon atoms; in particular synthetic triglycerides or preferably natural triglycerides, such as vegetable oils or oils of vegetable origin of the rapeseed oil, soybean oil, groundnut oil, butter oil, cottonseed oil, linseed oil, coconut oil, olive oil, palm oil, grapeseed oil, castor oil or copra oil type, or animal oils or oils of animal origin, for example fish oils, in particular fish oils comprising omega-3 fatty acids;
- esters of triglycerides of saturated or unsaturated fatty acids comprising at least 12 carbon atoms and preferably from 14 to 22 carbon atoms, in particular the methyl and ethyl esters thereof;
- aromatic petroleum fractions;
- aromatic solvents, for example anisole and toluene;
- terpene compounds, for example D-limonene and L-limonene;
- mixtures of dimethyl succinate/adipate/glutarate diesters;
- aliphatic hydrocarbons comprising at least 6 carbon atoms, for example isooctane, kerosene, gasoline, diesel, mineral oils, in particular liquid paraffin; lubricating oils; or
- mixtures thereof.

9. Composition according to Claims 1 to 8, wherein the nonpolar medium comprises, or even is, a mixture of triglycerides, for example a vegetable oil or oil of vegetable origin, selected from rapeseed oil, soybean oil, corn oil, castor oil, groundnut oil, butter oil, cottonseed oil, linseed oil, coconut oil, olive oil, palm oil, grapeseed oil, copra oil, and mixtures thereof.

10. Composition according to Claims 1 to 9, comprising between 0.5% and 40% by weight, for example between 1% and 20% by weight, preferably between 2% and 10% by weight, for example between 3% and 8% by weight, of copolymer, relative to the total weight of the composition.

**11.** Composition according to Claims 1 to 10, wherein the nonpolar medium is a vegetable oil or a mixture of vegetable oils and the emulsifier is a polyethylene glycol ester of fatty acids.

**12.** Composition according to Claims 1 to 11, comprising between 3% and 30% by weight of emulsifier relative to the total weight of the composition.

**13.** Process for preparing a composition according to one of Claims 1 to 12, selected from:

- a process (P1) comprising the following steps:

  (i) the nonpolar medium and the emulsifier are mixed; then
  (ii) the copolymer is added; then
  (iii) the compound to be dispersed is added; or

- a process (P2) comprising the following steps:

  (a) the nonpolar medium, the emulsifier and the compound to be dispersed are mixed; then
  (b) the copolymer, dissolved in the nonpolar medium, is added while hot; or

- a process (P3) comprising the following steps:

  (I) the nonpolar medium and the compound to be dispersed are mixed; then
  (II) the copolymer, dissolved in the nonpolar medium, and the emulsifier are added while hot.

**14.** Use of a composition according to one of Claims 1 to 12, for forming an emulsion of oil-in-water type by mixing the composition with an aqueous phase.

**15.** Concentrated composition comprising a copolymer of which the backbone is obtained by radical polymerization:

▪ of a fatty substance (A) comprising unsaturations selected from vegetable oils or oils of vegetable origin, selected from triglycerides of saturated or unsaturated fatty acids comprising at least 12 carbon atoms and preferably from 14 to 22 carbon atoms; esters of triglycerides of saturated or unsaturated fatty acids comprising at least 12 carbon atoms and preferably from 14 to 22 carbon atoms, and in particular the methyl and ethyl esters thereof; or animal oils or oils of animal origin, for example fish oils; or mixtures thereof; and
▪ of at least one monomer (B) comprising at least one function that can be polymerized by radical polymerization and that comprises at least one linear or branched alkyl chain comprising from 16 to 44 carbon atoms
▪ and an emulsifier, preferably selected from polyethylene glycol esters of fatty acids.

**16.** Concentrated composition according to Claim 15, with said copolymer being as defined in Claims 2 to 7.

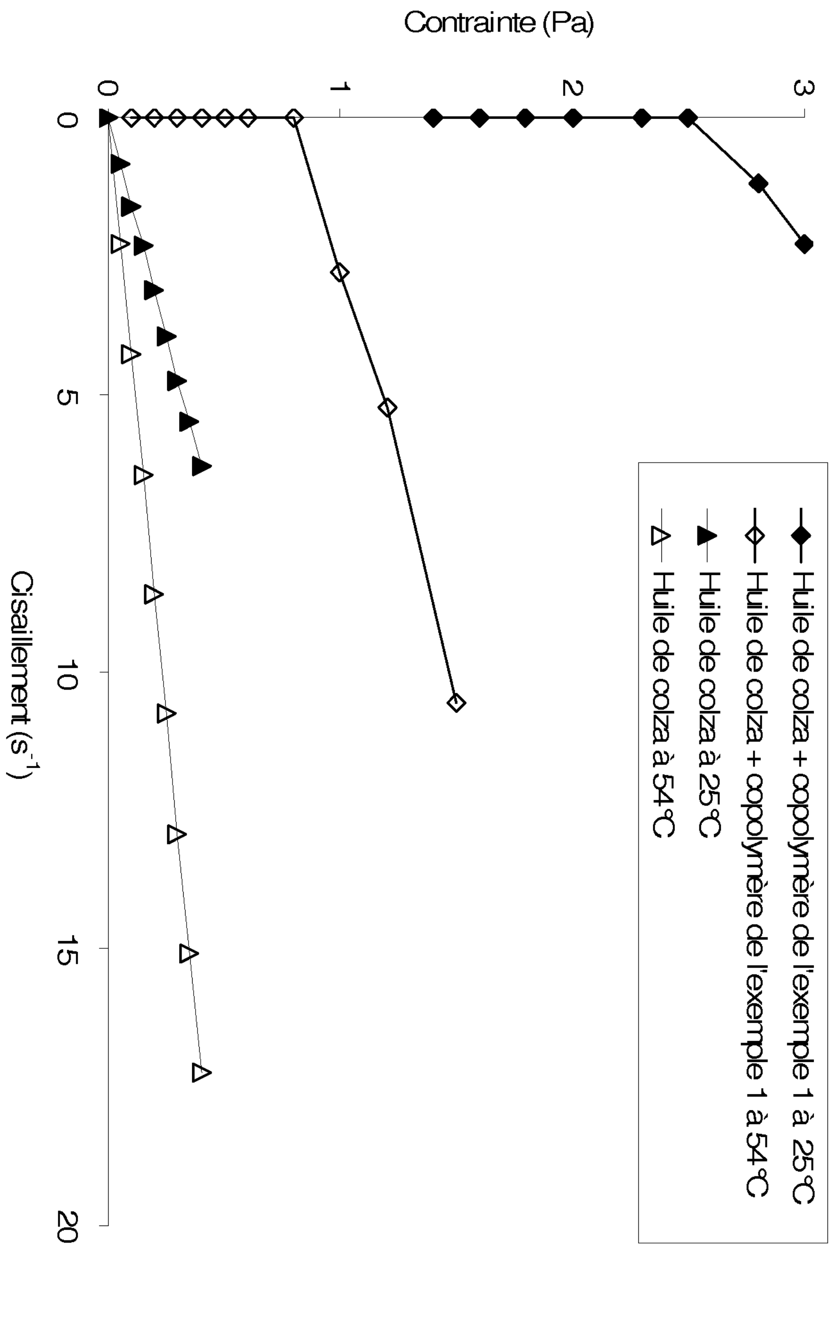
Contrainte (Pa)
0
1
2
3
0
5
10
15
20
Cisaillement (s-1)
Huile de colza + copolymère de l'exemple 1 à 25°C
Huile de colza + copolymère de l'exemple 1 à 54°C
Huile de colza à 25°C
Huile de colza à 54°C

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **HANSEN ; ALLAN F.M. BARTON.** Handbook of solubility parameters and other cohesion parameters. CRC Press Inc, 1983